# EUROPÄISCHE PATENTSCHRIFT

(11) **EP 3 040 101 B1**
(45) Veröffentlichungstag und Bekanntmachung des Hinweises auf die Patenterteilung: **22.02.2017**
(21) Anmeldenummer: 14200442.3
(22) Anmeldetag: 29.12.2014
(51) Int. Cl.: A61M 39/22, A61M 5/168, A61M 5/172, A61M 5/30, A61M 5/19, A61B 17/3203, A61M 3/02

(54) **Versorgungseinrichtung zur Erzeugung eines gepulsten Fluidstrahls, Applikationssystem mit Versorgungseinrichtung und computerlesbarer Speicher**
Supply device for generating a pulsed fluid jet, application system with a supply device and computer readable memory
Dispositif d'alimentation destiné à la fabrication d'un faisceau de liquide pulsé, système d'application doté d'un dispositif d'alimentation et mémoire adressable par ordinateur

(43) Veröffentlichungstag der Anmeldung: 06.07.2016
(73) Patentinhaber: Erbe Elektromedizin GmbH, 72072 Tübingen (DE)
(72) Erfinder: Fech, Andreas, 72072 Tübingen (DE); Fischer, Klaus, 72202 Nagold (DE); Blobel, Lars, 72119 Ammerbuch-Entringen (DE); Wandel, Waldemar, 72127 Kusterdingen (DE); Enderle, Markus D., 72070 Tübingen (DE)
(74) Vertreter: Bohnenberger, Johannes

(56) Entgegenhaltungen:
- EP-A1- 2 722 008
- EP-A1- 2 730 240
- WO-A1-2013/171311
- US-A1- 2010 160 897
- US-A1- 2013 144 207

## Beschreibung

Die vorliegende Erfindung betrifft eine Versorgungseinrichtung zur Erzeugung eines gepulsten Fluidstrahls, ein entsprechendes Applikationssystem sowie ein Steuerverfahren zum Betreiben der Versorgungseinrichtung.

Entsprechende Applikationsinstrumente, die dazu geeignet sind, Substanzen oder Suspensionen, insbesondere Zellen, in ein biologisches Gewebe einzubringen, sind bekannt. Beispielsweise beschreibt die US 2001/0027296 A1 ein Applikationsinstrument, das Zellen aus einem Gewebe gewinnen kann, diese aufbereitet und dann wieder in das Gewebe einbringt.

Die US 2013/0144207 beschreibt einen Mittel- und Hochdruck-Strahlgenerator, bei dem mindestens ein flexibler Behälter für eine Flüssigkeit verwendet werden kann. Dazu wird der flexible Behälter in einer Hochdruckkammer angeordnet. Zusätzlich ist in der Hochdruckkammer ein hydraulischer Sequenzer angeordnet, welcher dazu dient, gepulste Flüssigkeitsstrahlen zu erzeugen.

Die US 2010/0160897 zeigt eine Abgabeeinheit für die Abgabe eines therapeutischen Agens. Die Flüssigkeit kann über eine druckbeaufschlagte Flüssigkeitskartusche ausgebracht werden.

Das Instrument der US 2011/0282381 A1 basiert im Wesentlichen darauf, dass ein entsprechender Kanal im Gewebe zum Einbringen der Substanzen bereits vorhanden ist. Gegebenenfalls kann ein entsprechender Kanal mit einer Spitze gestochen werden. Das Bereitstellen eines entsprechenden Kanals führt zu einer erheblichen Schädigung des zu behandelnden Gewebes. Des Weiteren ist es mit dem beschriebenen Instrument sehr schwierig, eine weiträumige und homogene Verteilung der einzubringenden Substanz zu erzielen.

Aus der EP 2 722 008 ist ein Applikationssystem mit einer Versorgungseinrichtung bekannt, das dazu dient, Substanzen mittels eines Wasserstrahls in Gewebe einzubringen. Ein Problem dieses Applikatorsystems besteht darin, dass aufgrund der elastischen Dehnung des Applikators sowie der im System vorhandenen Toträume (= luftgefüllte Bereiche innerhalb der Fluidkanäle) ein Nachlaufen (Nachtropfen) des Fluids aus der Düsenöffnung zu beobachten ist. Da die Energiedichte des Strahls in der Nachlauf-Phase nicht ausreichend hoch ist, um in das Gewebe einzudringen, geht eine gewisse Menge der Suspension verloren. Des Weiteren ist es bei den bekannten Systemen notwendig, an den Ausgängen der Versorgungseinrichtung einen deutlich höheren Druck als an der Auslassdüse des Applikators anzulegen, so dass bei dem zu erwartenden Druckabfall innerhalb des Systems (z.B. aufgrund der Reibung) ein ausreichend hoher Druck an der Austrittsdüse auftritt. Dies kann die der Suspension beigemischten Zellen schädigen.

Ausgehend von diesem Stand der Technik ist es Aufgabe der vorliegenden Erfindung, eine Versorgungseinrichtung anzugeben, die eine effizientere Abgabe von Fluiden ermöglicht. Insbesondere soll die Versorgungseinrichtung dazu geeignet sein, Fluidstrahle mit einer hohen Austrittsgeschwindigkeit aus der Düse abzugeben, ohne dass die Suspension übermäßigen Belastungen ausgesetzt ist. Bei der Abgabe soll eine homogene Verteilung der Suspension erzielt werden.

Die Aufgabe wird durch den Gegenstand des Anspruchs 1 gelöst.

Insbesondere wird die Aufgabe durch eine Versorgungseinrichtung mit mindestens einem Auslass zum Anschluss eines Applikationsinstruments und mit einer Steuerung gelöst, wobei die Steuerung mindestens ein Ventil derart steuert, dass innerhalb eines Applikationszeitintervalls von weniger als 4 Sekunden:
a) ein erstes Fluid während mindestens eines ersten Förderintervalls mit einem ersten Druck in einer ersten Zulaufleitung;
b) ein zweites Fluid während eines zweiten, dem ersten Förderintervall zeitlich nachgelagerten Förderintervalls mit einem zweiten Druck in einer zweiten Zulaufleitung; und
c) das erste Fluid während mindestens eines dritten Förderintervalls mit einem dritten Druck in der ersten Zulaufleitung gefördert wird.

Ein Aspekt der Erfindung besteht darin, dass im Schritt c das erste Fluid als Treibmittel genutzt wird, um das zweite Fluid mit hohem Druck aus dem

Applikationsinstrument auszutreiben. Die Druckübergabe kann insofern sehr weit distal, beispielsweise nahe an der Düse, erfolgen. Hierdurch wird sichergestellt, dass das zweite Fluid einem Druckniveau ausgesetzt wird, das im Wesentlichen dem entspricht, mit dem das Fluid aus der Düse austritt. Das Aufbringen eines höheren Druckwerts zur Kompensation von Verlusten ist hinsichtlich des zweiten Fluids nicht notwendig. Erfindungsgemäß ist es also möglich, Zellen mit einer hohen Überlebensrate in tiefe Gewebeschichten einzubringen. Erfindungsgemäß ist es möglich, Druckpulse zum Antreiben des ersten und/oder zweiten Fluids zu erzeugen.

In einer Ausführungsform ist der erste Druck im ersten Förderintervall deutlich größer als der zweite Druck. Des Weiteren ist der erste Druck vorzugsweise deutlich größer als der dritte Druck. In einer Ausführungsform kann die Förderung des ersten Fluids im ersten Förderintervall dazu genutzt werden, um einen Kanal im Gewebe für das Einbringen des zweiten Fluids zu schaffen (Kanal-Eröffnungsvorgang). Das Fördern des zweiten Fluids während des zweiten Förderintervalls kann dazu genutzt werden, um einen vorhandenen Speicher oder ein Reservoir aufzufüllen, wobei dann wiederum im dritten Förderintervall das in dem Reservoir gespeicherte Fluid ausgetrieben wird. Maßgeblich für das erfolgreiche Einbringen der Suspension sind der erste und der dritte Druck, wobei der dritte Druck darüber entscheidet, wie tief das zweite Fluid in das Gewebe eindringt.

Ein Applikationszeitintervall kann weniger als 4 Sekunden dauern. In einer Ausführungsform dauert das Applikationszeitintervall weniger als 3 Sekunden oder sogar weniger als 2 Sekunden. Erfindungsgemäß kann ein Applikationszeitintervall als ein Zeitraum verstanden werden, der mit der Abgabe des ersten Fluids zum Schneiden eines Kanals beginnt und mit der letzten Abgabe eines Fluids vor einem erneuten Kanal-Schneidevorgang endet. Ein Applikationszeitintervall umfasst also genau einen Schneidvorgang und mindestens einen Abgabevorgang, bei dem eine Suspension in das Gewebe eingebracht wird.

Die Versorgungseinrichtung kann mindestens eine Pumpe umfassen, die vorzugsweise durch die Steuerung derart gesteuert wird, dass ein im Wesentlichen konstanter Volumenfluss des ersten Fluids erzielt wird. Erfindungsgemäß ist es möglich, den ersten Druck und/oder den zweiten Druck und/oder den dritten Druck durch die Vorgabe einer bestimmten Fördergeschwindigkeit der Pumpe vorzugeben. In mindestens einer Ausführungsform wird der Volumenfluss der Pumpe jedoch über den Verlauf eines Applikationszeitintervalls im Wesentlichen konstant gehalten. Eine Druckregelung kann in diesem Fall über ein/das Ventil, insbesondere ein Stellventil, erfolgen.

In einer Ausführungsform umfasst ein Applikationszeitintervall mindestens eine Abgabephase und mindestens eine Bypassphase. Erfindungsgemäß kann eine Abgabephase so definiert sein, dass hierbei ein Fluid durch die Versorgungseinrichtung an einem Auslass so abgegeben wird, dass dies mittels des Applikationsinstruments appliziert werden kann. Eine Bypassphase kann erfindungsgemäß so verstanden werden, dass hier das Fluid an dem Applikationsinstrument vorbei-, vorzugsweise ab-, geführt wird.

Um eine schnelle Reaktion der Versorgungseinrichtung zu erreichen ist es vorteilhaft, wenn die Pumpe bei im Wesentlichen konstantem Volumenfluss fördert. Eine schnell veränderbare Druckeinstellung kann durch das gezielte Abführen von Fluid über eine Bypassleitung bzw. Bypasszweig erfolgen.

In einer Ausführungsform umfasst die Versorgungseinrichtung eine Bypassleitung zum Abführen des Fluids, wobei die Bypassleitung vorzugsweise ein Drosselelement, insbesondere ein Drosselventil umfasst. Die Steuerung kann mindestens ein erstes Ventil, insbesondere ein 3/2-Wegeventil, derart steuern, dass zur (gepulsten) Abgabe des ersten Fluids an dem Auslass abwechselnd eine fluide Verbindung zwischen Pumpe und einer Druckleitung in einer Abgabephase und zwischen Pumpe und Bypassleitung in einer/der Bypassphase hergestellt wird. Das Vorsehen der Bypassphase ermöglicht es in sehr effizienter Weise, eine gepulste Abgabe des Fluids umzusetzen. Im Endeffekt kann das erste Ventil so gesteuert werden, dass zeitweise eine Abführung über die Bypassleitung und zeitweise eine Abgabe über den Auslass erfolgt. Durch diese Steuerstrategie werden sehr steile Druckflanken beim Fördern des Fluids erzielt, so dass das abgegebene Fluid in kürzester Zeit das gewünschte Energieniveau erreicht.

Ebenso kann diese Steuerstrategie dazu verwendet werden, um in kürzester Zeit ein bestehendes Druckniveau wieder abzubauen, so dass neben der Anstiegszeit auch die Abfallszeit minimiert wird.

Die Steuerung kann das mindestens eine Ventil derart steuern, dass innerhalb des Applikationszeitintervalls genau eine Abgabephase und mindestens eine Bypassphase implementiert werden. Erfindungsgemäß ist es möglich, die genannten Schritte a-c mit einer Abgabephase umzusetzen. In einer bevorzugten Ausführungsform wird für jeden Schritt a und für jeden Schritt c eine Abgabephase durch die Steuerung implementiert, der vorzugsweise eine Bypassphase vorausgeht.

Das mindestens eine erste Ventil kann ein elektrisches Ventil sein, das derart angeordnet und ausgebildet ist, dass in einer bestromten Phase eine fluide Verbindung zwischen Pumpe und Druckleitung besteht. Im Endeffekt führt also die Bestromung des mindestens einen ersten Ventils zu einer Abgabephase. Diese Anordnung hat mehrere Vorteile. Zum einen muss das Ventil nur während der Aktivierung zur Abgabe einer Pulsfolge kurzzeitig mit Strom beaufschlagt werden. Zum anderen steht ein durch den Bypasszweig eingestelltes Druckniveau bereits beim ersten Abgeben eines Pulses zur Verfügung. Dies setzt natürlich voraus, dass an der Bypassleitung ein entsprechendes Ventil, insbesondere ein Drosselventil, vorgesehen ist.

Grundsätzlich ist es möglich, dass das erste Ventil invertiert eingebaut wird, so dass in der stromlosen Phase die Druckleitung in fluider Verbindung mit der Pumpe steht. In diesem Fall kann eine gekoppelte Ansteuerung der Pumpe und des Ventils vorteilhaft sein. Beispielsweise kann beim Aktivieren der Pumpe ein Schaltsignal an das Ventil gesendet werden, um das Ventil zu schließen, bevor der erste Puls abgegeben wird.

Wie bereits erläutert, kann die Bypassleitung in einer Ausführungsform mit einem Drosselelement versehen werden. Das Drosselelement kann derart eingestellt sein, dass an dem mindestens einen ersten Ventil ein Bypassdruck anliegt, der größer ist als der dritte Druck, insbesondere um mindestens 50% oder um mindestens 100% des dritten Drucks. Zur Erzeugung eines bestimmten Pulses mit einem Druckniveau pmax kann es vorteilhaft sein, innerhalb der Versorgungseinrichtung einen deutlich höheren Druck, z.B. einen Überdruck p'max, einzustellen, so dass bei der Öffnung des Ventils in der Versorgungseinrichtung oder auf der proximalen Seite des Applikationsinstruments zunächst eine Druckwelle abgegeben wird, die über dem gewünschten Druck liegt. Bei der Ausbreitung dieser Druckwelle kommt es zwangsläufig in jedem System zu einer Dämpfung, so dass letztendlich am Effektor, beispielsweise der Düse, der gewünschte Druck ankommt. Dieser zu hohe Druck kann in geeigneter Weise unter Verwendung der Bypassleitung mit dem beschriebenen Drosselelement erreicht werden. In einer Ausführungsform arbeitet die Pumpe, obwohl kein Fluid über den Auslass abgegeben wird, gegen das Drosselelement, so dass sich in der Versorgungseinrichtung ein Druck aufbaut. Bei diesem Druck kann es sich um den Bypassdruck handeln. Vorzugsweise wird der Bypassdruck so eingestellt, dass er sich relativ schnell über den Auslass abbaut. Nach dem Propagieren der bereits beschriebenen Druckwelle kann die Leistung der Pumpe maßgeblich dafür sein, mit welchem Druck das Fluid letztendlich abgegeben wird.

Erfindungsgemäß kann ein Druckspeicher vorgesehen werden, der vorzugsweise zur Speicherung des Bypassdrucks angeordnet ist. Der Druckspeicher kann so dimensioniert sein, dass sich der Bypassdruck in der Zeit abbaut, die die Druckwelle bis zum Erreichen des Effektors benötigt.

Zusätzlich oder alternativ kann das Drosselelement derart eingestellt sein, dass ein Bypassdruck anliegt, der größer ist als der erste Druck, insbesondere um 5% oder um mindestens 10% des ersten Drucks. Vorzugsweise wird die oben beschriebene Maßnahme dazu eingesetzt, um eine möglichst pulsförmige Druckflanke bei der Abgabe während des ersten Zeitintervalls (Schritt a) zu erzielen.

Zusätzlich oder alternativ kann diese Maßnahme jedoch auch eingesetzt werden, um eine im Wesentlichen pulsförmige Druckflanke während der Fluidgabe im dritten Zeitintervall (Schritt c) zu erreichen. In einer Ausführungsform kann die Steuerung das Drosselelement unmittelbar oder ein dem Drosselelement vor- oder nachgeschaltetes Ventil so einstellen, dass der Bypassdruck in Abhängigkeit von der zu erwartenden Phase (Schritt a oder Schritt c) variiert. Insofern kann dafür Sorge getragen werden, dass sowohl während des ersten Förderintervalls wie auch während des dritten Förderintervalls eine steile Druckflanke erreicht wird.

Zusätzlich oder statt der bereits beschriebenen Bypassleitung kann die Vorsorgeeinrichtung eine (weitere) Bypassleitung umfassen.

Diese weitere Bypassleitung kann über ein (drittes) Ventil mit der Druckleitung in fluider Verbindung stehen bzw. in fluide Verbindung bringbar sein, um die Druckleitung in einer Entlüftungsphase zu entlüften. Bei dem dritten Ventil kann es sich um ein 2/2-Wegeventil handeln. Die weitere Bypassleitung kann dazu eingesetzt werden, die Druckleitung zeitnah zu entlüften. Insofern kann nach dem Aufbauen eines vorgegebenen Drucks dieser sehr schnell wieder abgebaut werden. Das dritte Ventil kann beispielsweise derart gesteuert werden, dass am Ende des ersten und/oder dritten Förderintervalls eine entsprechende Entlüftung stattfindet.

In einer Ausführungsform steuert die Steuerung das dritte Ventil derart, dass innerhalb des Applikationszeitintervalls mindestens eine Entlüftungsphase implementiert wird.

Die eingangs genannte Aufgabe wird des Weiteren durch ein Applikationssystem gelöst.

Das Applikationssystem kann ein Applikationsinstrument und eine Versorgungseinrichtung wie diese bereits beschrieben wurde umfassen.

Seitens des Applikationssystems ergeben sich ähnliche Vorteile, wie diese bereits in Verbindung mit der Versorgungseinrichtung beschrieben wurden. In einer Ausführungsform umfasst das Applikationssystem mindestens ein Ventil, das in oder an dem Applikationsinstrument angeordnet ist. Dieses mindestens eine Ventil wird vorzugsweise von der Steuerung der Versorgungseinrichtung gesteuert.

Erfindungsgemäß wird es angestrebt, zumindest einige der zur Herstellung der Pulsform notwendigen Steuerventile möglichst nahe am Applikationsinstrument oder in diesem anzuordnen, so dass sehr steile Pulsflanken beim Aufbau und/oder Abbau des Drucks erzielt werden. Insgesamt fördert die instrumentennahe, insbesondere düsennahe, Anordnung der Ventile die Reaktionsfreudigkeit des Applikationssystems, so dass eine jede gewünschte Druckführung implementiert werden kann.

Die eingangs genannte Aufgabe wird des Weiteren durch ein Steuerverfahren zum Betreiben einer Versorgungseinrichtung gelöst. Vorzugsweise handelt es sich bei In einer Ausführungsform umfasst das Steuerverfahren die folgenden Schritte:a) Aktivieren einer ersten Fluidquelle derart, dass in einer Bypassphase ein Bypassdruck aufgebaut wird;b) Öffnen mindestens eines Ventils derart, dass in einer Abgabephase ein erstes Fluid mit dem Bypassdruck in eine Druckleitung abgegeben wird;c) Betreiben der Fluidquelle derart, dass nach der Bypassphase und während der Abgabephase ein Druck in der Druckleitung vorliegt, der geringer, insbesondere um mindestens 5% oder mindestens 6% geringer, ist als der Bypassdruck.

Ein erfindungswesentlicher Aspekt dieses Steuerverfahrens beste gabe eines bestimmten Druckimpulses einen Überdruck p'max, wie dies bereits beschrieben wurde, aufzubauen, um einer Abschwächung bei der Ausbreitung des Druckimpulses vorzubeugen. Erfindungsgemäß gibt es also zwei Stellgrößen zur optimalen Betreibung der Versorgungseinrichtung. Hierbei handelt es sich zum einen um den Bypassdruck und zum anderen um die Förderleistung der Fluidquelle, bei der es sich vorzugsweise um eine Pumpe handelt.

In einer Ausführungsform umfasst das Steuerverfahren ein Einschalten mindestens eines Ventils derart, dass das erste Fluid zeitweise ein zweites Fluid antreibt.

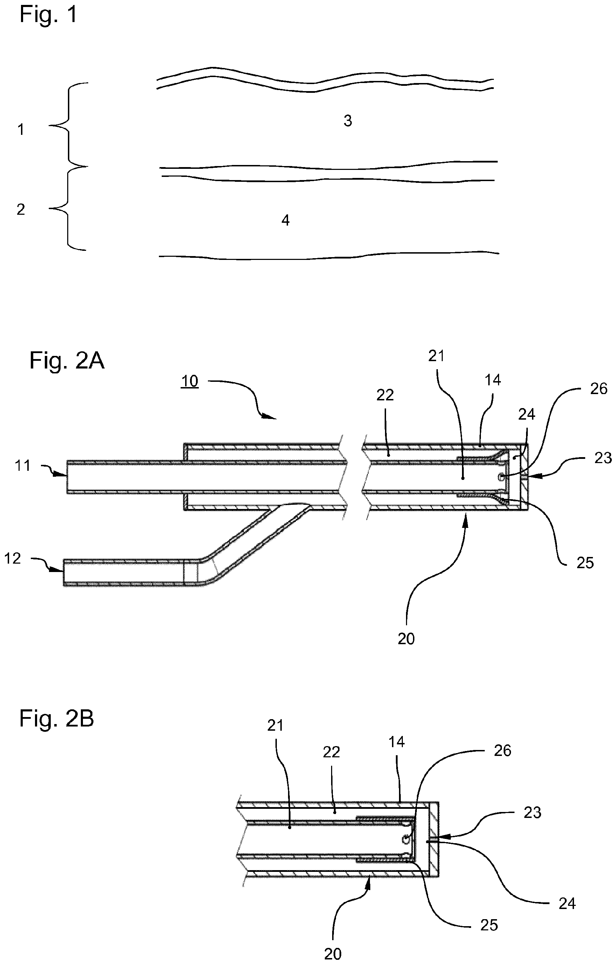

Die eingangs genannte Aufgabe wird des Weiteren durch einen computerlesbaren Speicher mit Instruktionen zur Implementierung des beschriebenen Verfahrens gelöst, wenn die Instruktionen eines Steuerverfahrens zum Betreiben einer Versorgungsvorrichtung gemäss au Anspruchs 11 geführt d von mehreren Ausführungsbeispielen beschrieben. Hierbei zeigt:Fig. 1 eine schematische Darstellung des Schichtaufbaus eines Hohlorgans;
- Fig. 2A: eine schematische Darstellung eines Applikationsinstruments gemäß einer ersten Ausführungsform (nur Wechselventil) mit Fluidfluss aus einem Innenzufuhrkanal;
- Fig. 2B: eine schematische Darstellung des Applikationsinstruments gemäß der ersten Ausführungsform mit Fluidfluss aus einem Außenzufuhrkanal;
- Fig. 3A: eine schematische Darstellung eines Applikationsinstruments gemäß einer zweiten Ausführungsform (Wechselventil mit distalem Rückschlagventil) mit Fluidfluss aus dem Innenzufuhrkanal;
- Fig. 3B: eine schematische Darstellung des Applikationsinstruments gemäß der zweiten Ausführungsform mit Fluidfluss aus dem Außenzufuhrkanal;
- Fig. 4A: eine schematische Darstellung eines Instrumentenkopfs gemäß einer dritten Ausführungsform (elastische Düse) mit Fluidfluss aus dem Außenzufuhrkanal;
- Fig. 4B: eine schematische Darstellung des Instrumentenkopfs gemäß der dritten Ausführungsform mit Fluidfluss aus dem Innenzufuhrkanal;
- Fig. 5: einen Instrumentenkopf einer vierten Ausführungsform (ebenfalls elastische Düse mit Zellsuspensionszufuhr durch den Außenzufuhrkanal);
- Fig. 6A: eine schematische Darstellung eines Applikationsinstruments gemäß einer fünften Ausführungsform (Entlüftungseinrichtung) mit Fluidfluss aus dem Außenzufuhrkanal;
- Fig. 6B: eine schematische Darstellung des Applikationsinstruments gemäß der fünften Ausführungsform mit Fluidfluss aus dem Innenzufuhrkanal;
- Fig. 7A: eine schematische Darstellung eines Applikationsinstruments gemäß einer sechsten Ausführungsform (Wechselventil mit proximalem Rückschlagventil aus einem elastischen Element) mit Fluidfluss durch den Außenzufuhrkanal;
- Fig. 7B: eine Detailansicht des elastischen Elements aus Fig. 7A;
- Fig. 8A: eine schematische Darstellung einer ersten alternativen Ausführungsform eines Wechselventils an dem Innenzufuhrkanal;
- Fig. 8B: eine schematische Darstellung einer zweiten alternativen Ausführungsform eines Wechselventils an dem Innenzufuhrkanal;
- Fig. 8C: eine schematische Darstellung einer dritten alternativen Ausführungsform des Ventilsitzes des Wechselventils an dem Innenzufuhrkanal;
- Fig. 9A: eine schematische Darstellung eines Applikationsinstruments gemäß einer siebten Ausführungsform;
- Fig. 9B: eine Detailansicht des Instrumentenkopfs gemäß Fig. 9A;
- Fig. 10: eine schematische Darstellung einer Versorgungseinrichtung gemäß einem ersten Ausführungsbeispiel, wobei alle Steuerventile in die Versorgungseinrichtung integriert sind;
- Fig. 11: eine schematische Darstellung einer Versorgungseinrichtung gemäß einem zweiten Ausführungsbeispiel, wobei ein Steuerventil in das Applikationsinstrument integriert ist;
- Fig. 12: eine schematische Darstellung eines ersten alternativen Aufbaus zur Erzeugung eines gepulsten Wasserstrahls;
- Fig. 13: eine schematische Darstellung eines zweiten alternativen Aufbaus zur Erzeugung eines gepulsten Wasserstrahls;
- Fig. 14: eine schematische Darstellung eines dritten alternativen Aufbaus zur Erzeugung eines gepulsten Wasserstrahls;
- Fig. 15: eine schematische Darstellung eines vierten alternativen Aufbaus zur Erzeugung eines gepulsten Wasserstrahls;
- Fig. 16: eine schematische Darstellung eines fünften alternativen Aufbaus zur Erzeugung eines gepulsten Wasserstrahls;
- Fig. 17: einen durch eine Versorgungseinrichtung generierten Druckverlauf gemäß einem ersten Steueralgorithmus;
- Fig. 18: einen durch eine Versorgungseinrichtung generierten Druckverlauf gemäß einem zweiten Steueralgorithmus;
- Fig. 19: den Druckverlauf gemäß Fig. 18 mit zusätzlich angezeigten Bypass- und Entlüftungsphasen.

In der nachfolgenden Beschreibung werden für gleiche und gleich wirkende Teile dieselben Bezugsziffern verwendet.

Fig. 1 zeigt eine schematische Darstellung des Schichtaufbaus eines Hohlorgans der ableitenden Harnwege. Wesentliche Gewebeschichten sind die Mucosa 1 und die Muscularis 2. Der Harnweg befindet sich in der Darstellung ganz oben. Danach folgt ein Epithel, das wiederum von der Lamina propria 3 gefolgt ist. Danach sind die Längsmuskel und Zirkulärmuskel 4 dargestellt. Das erfindungsgemäße Applikationssystem kann eingesetzt werden, um eine schnellere Regeneration eines Sphinkter-Defekts des dargestellten Harntrakts zu gewährleisten.

Das Applikationssystem ermöglicht eine tissue-engineering-basierte Therapie, bei der eine Suspension, beispielsweise Zellen in einem Nährmedium, in den Urethra-Schließmuskel durch mehrere vorgelagerte Gewebeschichten hindurch mit ausreichend hoher Überlebensrate der Zellen transportiert und dort möglichst verlustarm deponiert werden. Idealerweise wird hierbei eine Schädigung des noch intakten Schließmuskelgewebes verhindert. Der Zirkulärmuskel 4 aus Fig. 1 stellt daher ein mögliches Zielgewebe für das erfindungsgemäße Applikationssystem dar, wobei der applizierte Wasserstrahl zunächst die Mucosa 1 perforieren muss, um die Substanz zur Muscularis 2 zu transportieren.

Es gibt zahlreiche alternative Einsatzgebiete für das erfindungsgemäße Applikationssystem, beispielsweise Gallenwege, Gastrointestinalwände, Gefäßwände, Bronchialwände usw.

Fig. 2 zeigt ein erstes Ausführungsbeispiel eines erfindungsgemäßen Applikationsinstruments 10. Ein wesentlicher Bestandteil des Applikationsinstruments 10 ist der Sondenschaft 14, der vorzugsweise zumindest teilweise elastisch ist und proximal einen Instrumentenkopf 20 aufweist. Dieser Instrumentenkopf 20 hat eine Düse 23 zur Abgabe von Fluiden. Bei den Fluiden kann es sich um eine Kochsalzlösung handeln oder um die bereits erwähnte Suspension mit Zellanteilen. Um die Fluide zuführen zu können, ist im Lumen des Sondenschafts 14 koaxial ein Innenzufuhrkanal 21 angeordnet. Der Außenbereich des Innenzufuhrkanals 21 bildet den Außenzufuhrkanal 22, der den Innenzufuhrkanal 21 umgibt. Der Innenzufuhrkanal 21 steht in dem Zustand, wie er in Fig. 2A gezeigt ist, über Seitenöffnungen 26 in fluider Verbindung mit einem Distalspeicher 24. Ein an der distalen Spitze des Innenzufuhrkanals 21 angeordnetes Wechselventil 25 ermöglicht das Austreten eines ersten Fluids aus dem Innenkanal 21 in den Distalspeicher 24 und verschließt eine fluide Verbindung zwischen dem Distalspeicher 24 und dem Außenzufuhrkanal 22.

Der Innenzufuhrkanal 21 wird über einen ersten Zulauf 11, und der Außenzufuhrkanal 22 über einen zweiten Zulauf 12 mit dem ersten bzw. zweiten Fluid versorgt.

Fig. 2B zeigt eine Detailansicht des Instrumentenkopfs 20 aus Fig. 2A. Im Gegensatz zu der Darstellung gemäß Fig. 2A verschließt das Wechselventil 25 in Fig. 2B die Seitenöffnungen 26, so dass eine unmittelbare fluide Verbindung zwischen dem Distalspeicher 24 und dem Außenzufuhrkanal 22 besteht.

Ein Aspekt der vorliegenden Erfindung besteht darin, die zugeführten Fluide in einer annähernd perfekten Pulsform über eine Austrittsöffnung 23, die Düse 23 abzugeben. Der erfindungsgemäße Instrumentenkopf 20 ermöglicht es bei relativ niedrigen Drücken, Fluidpulse abzugeben, mit denen die Fluide in geeigneter Weise in das Zielgewebe eindringen können. Aufgrund der effizienten Nutzung der vorhandenen Drücke wird die Zellsuspension bei der Applikation "geschont". Ein weiterer Aspekt der Erfindung besteht darin, mittels der Steuerung der Pulse die Fluide, insbesondere die Zellsuspension in unterschiedliche Ebenen des Zielgewebes einzubringen. Aufgrund der effizienten Nutzung der vorhandenen Drücke kann die Zellsuspension bei der Applikation "geschont an das Zielgewebe insbesondere an verschiedene Stellen eingebracht werden.

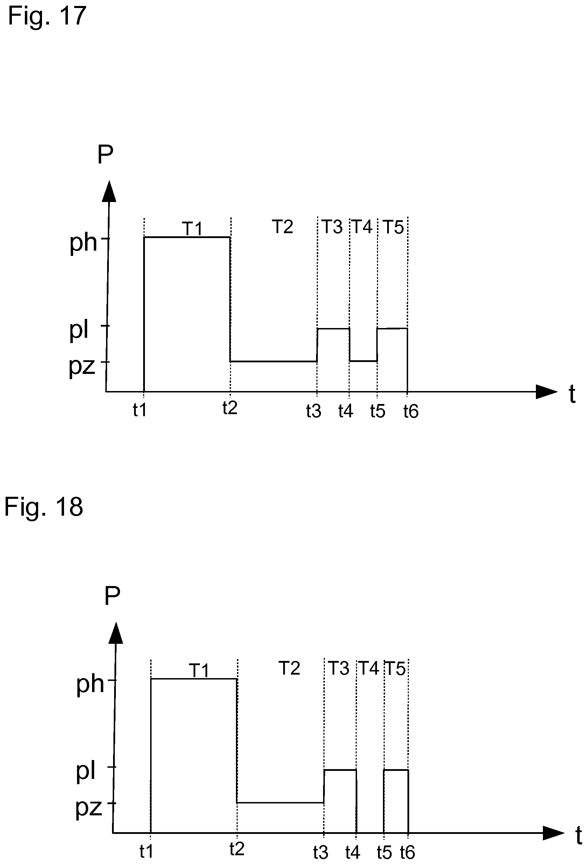

Für die effektive Einbringung der Suspension wird in einem Applikationszeitintervall, beispielsweise wie in Fig. 17 gezeigt, das erste Fluid zunächst in einem ersten Zeitintervall T1 mit einem hohen Druck ph gefördert. In diesem ersten Zeitintervall T1 befindet sich der Instrumentenkopf 20 in dem Zustand, wie er in Fig. 2A gezeigt ist. Das erste Fluid tritt aus dem Innenzufuhrkanal 21 aus, füllt den Distalspeicher 24 und wird über die Düse 23 mit einem definierten Düsendurchmesser abgegeben. Das erste Fluid trifft also mit einer hohen kinetischen Energie auf das Gewebe und kann genutzt werden, um einen Einbringungskanal zu schaffen. In einem nachfolgenden zweiten Zeitintervall T2 wird das zweite Fluid mit einem sehr niedrigen Druck pz angetrieben, so dass sich der Distalspeicher 24 mit dem zweiten Fluid - also der Suspension - füllt. In dieser Phase kann der Instrumentenkopf 20 den Zustand einnehmen, wie dieser in Fig. 2B gezeigt ist. Das Wechselventil 25 verschließt den Innenzufuhrkanal 21, so dass das Nachlaufen des ersten Fluids verhindert wird. Nach der Füllung des Distalspeichers 24 wird das erste Fluid in dem dritten Zeitintervall T3 mit einem Druck pl gefördert. Dieser Druck pl ist vorzugsweise deutlich geringer als der hohe Druck ph, so dass eine schonende Ausbringung der Suspension erfolgt. In dem dritten Zeitintervall T3 nimmt der Instrumentenkopf 20 wieder den Zustand ein, wie dieser in Fig. 2A gezeigt ist. Das erste Fluid dringt in den Distalspeicher 24 ein und verdrängt das zweite Fluid. Das erste Fluid ist also Treibmittel und dient zum Austreiben des zweiten Fluids bei gegebenem Druck pz. Je nach Ausgestaltung kann der Distalspeicher 24 in einem Applikationszeitintervall ein weiteres Mal gefüllt werden (vgl. viertes Zeitintervall T4) und die Suspension ein weiteres Mal abgegeben werden (vgl. Zeitintervall T5).

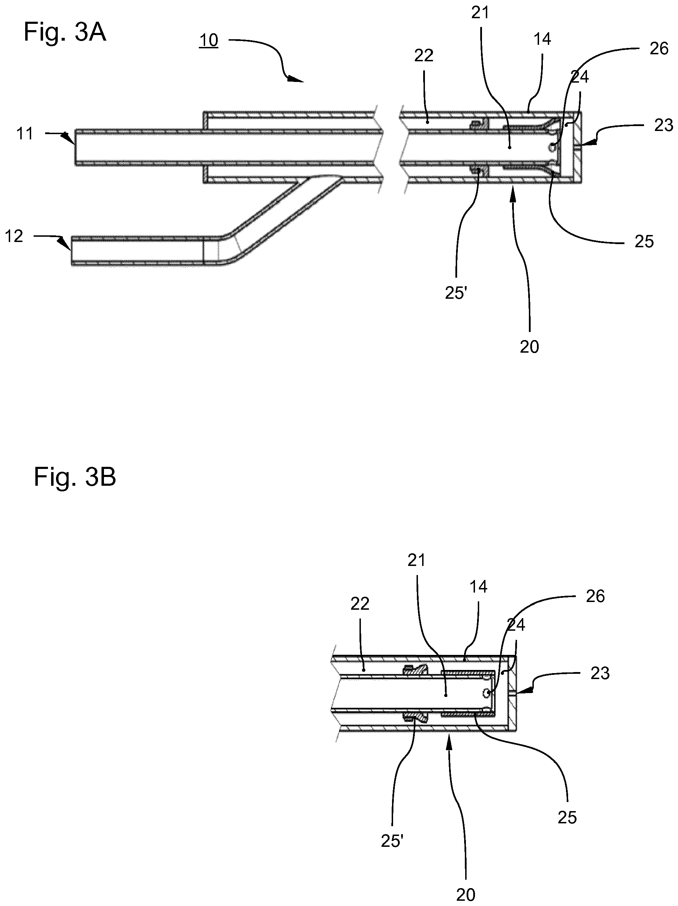

Fig. 3A zeigt eine weitere Ausführungsform des erfindungsgemäßen Applikationsinstruments 10. Gegenüber der Ausführungsform aus den Fig. 2A und 2B ist in der Ausführungsform gemäß Fig. 3A ein weiteres Ventil im Instrumentenkopf 20 vorgesehen. Das weitere Ventil, ein Rückschlagventil 25', befindet sich ebenso wie das Wechselventil 25, im Außenzufuhrkanal 22. Gegenüber dem Wechselventil 25 ist das Rückschlagventil 25' weniger nahe an der distalen Spitze des Applikationsinstruments 10 angeordnet. Bei dem Rückschlagventil 25' handelt es sich um eine Gummilippe, die im druckfreien Zustand den Außenzufuhrkanal 22 vollständig verschließt. Fig. 3A zeigt einen entsprechenden druckfreien Zustand, bei dem das erste Fluid gefördert und über die Düse 23 ausgegeben wird.

Bei einer Förderung des zweiten Fluids über den zweiten Zulauf in dem Außenzufuhrkanal 22 öffnet sich das Rückschlagventil 25' und das Wechselventil 25 verschließt, wie bereits erläutert, die Seitenöffnungen 26. Ein entsprechender Zustand ist in Fig. 3B gezeigt. In diesem Zustand kann der Distalspeicher 24 befüllt werden. Sobald der Fluidfluss in dem Außenzufuhrkanal 22 zum Erliegen kommt, schließt sich das Rückschlagventil 25'. Insofern wird ein Nachlaufen des zweiten Fluids verhindert. Übersteigt der Druck in dem Innenzufuhrkanal 21 den Druck des Distalspeichers 24, öffnet sich das Wechselventil 25. Dies kann dazu führen, dass bei dem gepulsten Strahl energetisch eine sehr steile Anfangsflanke erzeugt werden kann.

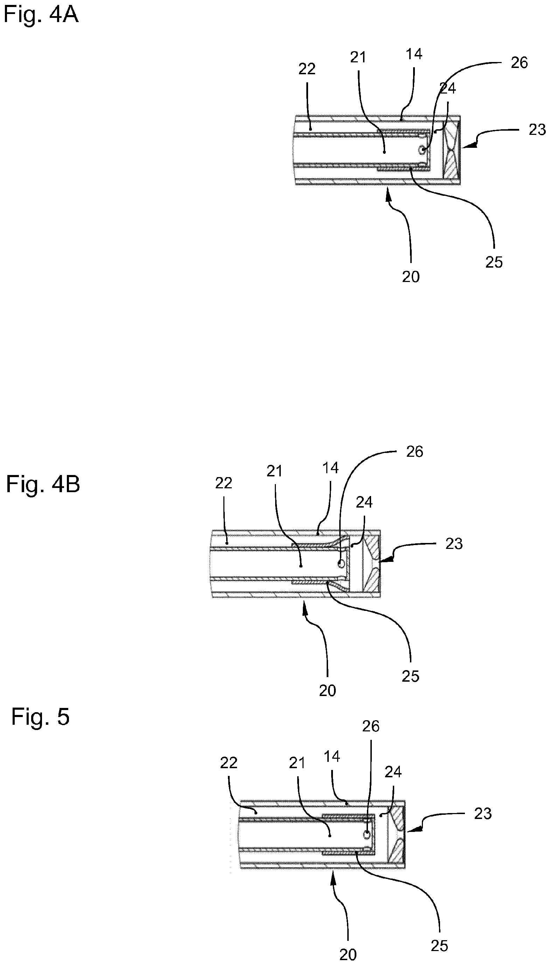

Die beschriebene Ausführungsform lässt sich in besonders vorteilhafter Weise mit einer elastischen Düse 23 einsetzen, wie diese exemplarisch in der Fig. 4A und 4B gezeigt ist. Die elastische Düse 23 kann aber auch unabhängig von einem vorhandenen oder nicht vorhandenen Rückschlagventil 25' Vorteile haben.

Die elastische Düse 23 gemäß Fig. 4A ist im druckfreien Zustand geschlossen. Hierzu wird der elastische Düsenkörper derart in das Applikationsinstrument 10 eingebaut, dass eine definierte Pressung radial auf den Düsenkörper wirkt und im Ausgangszustand die Düsenöffnung verschließt. Steigt der Druck proximal des Düsenkörpers im Distalspeicher 24, beispielsweise infolge der Zufuhr des zweiten Fluids, so wird der Düsenkörper zunächst geringfügig nach außen gewölbt, ohne dass die Düsenöffnung geöffnet wird (nicht gezeigt). Somit kann eine definierte Volumenmenge in dem Distalspeicher 24 vordosiert werden. Anschließend kann das vordosierte Volumen wie bereits beschrieben mit einem nachfolgenden Hochdruckpuls (vgl. drittes oder fünftes Zeitintervall T3, T5) in den im Gewebe geöffneten Kanal eingetragen werden. Der in diesem Ausführungsbeispiel beschriebene Düsenkörper besitzt eine umlaufende Lippe, die sich in Radialrichtung verjüngt. Der Düsenkörper kann auch zweigeteilt aufgebaut werden. Beispielsweise kann die umlaufende Lippe aus einem elastischen Material bestehen, während sie an ihrer Basis von einem Träger aus einem harten Material umfasst wird. Das Verhalten der elastischen Düse 23, insbesondere dessen Dehnung, in der Vordosierungs- bzw. Befüllungsphase, hängt entscheidend von der Materialwahl und dem Ausmaß der Pressung im eingebauten Zustand ab. Erfindungsgemäß ist die elastische Düse 23 derart ausgestaltet, dass eine ausreichende Dehnung und somit die Aufnahme eines Vordosierungsvolumens im Distalspeicher 24 erreicht werden kann, ohne dass ein hoher Druck, beispielsweise höher als 20 bar erforderlich ist. Weiterhin ist die elastische Düse so ausgestaltet, dass im offenen Zustand die Düsenöffnung 23 gerade so groß ist, das eine Düsenwirkung, also eine ausreichende Beschleunigung des Fluids, erzielt werden kann.

Die elastische Düse 23 kann erfindungsgemäß dazu eingesetzt werden, einen Nachlauf des Fluids nach der Applikation des ersten und/oder zweiten Fluids zu verhindern. Gleichzeitig speichert sie bei entsprechender Befüllung des Distalspeichers 24 einen gewissen Vordruck, der abgerufen werden kann. Weiterhin minimiert die elastische Düse 23 die Gefahr des Verstopfens des Applikationsinstruments 10. Die Verstopfung führt bei der erfindungsgemäßen Ausgestaltung lediglich zu einem Druckanstieg, der wiederum eine Dehnung der Düse 23 derart veranlasst, dass Verunreinigungspartikel passieren können.

Fig. 4B zeigt den Instrumentenkopf 20 mit geöffneter elastischer Düse 23, beispielsweise während des dritten Zeitintervalls T3.

Fig. 5 zeigt eine alternative Ausführungsform des Instrumentenkopfs gemäß Fig. 4A und 4B. Hier dient der Außenzufuhrkanal 22 zur Zufuhr des ersten Fluids und der Innenzufuhrkanal 21 zur Zufuhr des zweiten Fluids. Der in Fig. 5 gezeigte Zustand tritt beispielsweise im ersten Zeitintervall T1 auf, wenn das erste Fluid zur Schaffung eines Gewebekanals verwendet wird. Auch bei den anderen bereits beschriebenen Ausführungsformen kann erfindungsgemäß der Innenzufuhrkanal 21 für das zweite Fluid, und der Außenzufuhrkanal 22 für das erste Fluid verwendet werden.

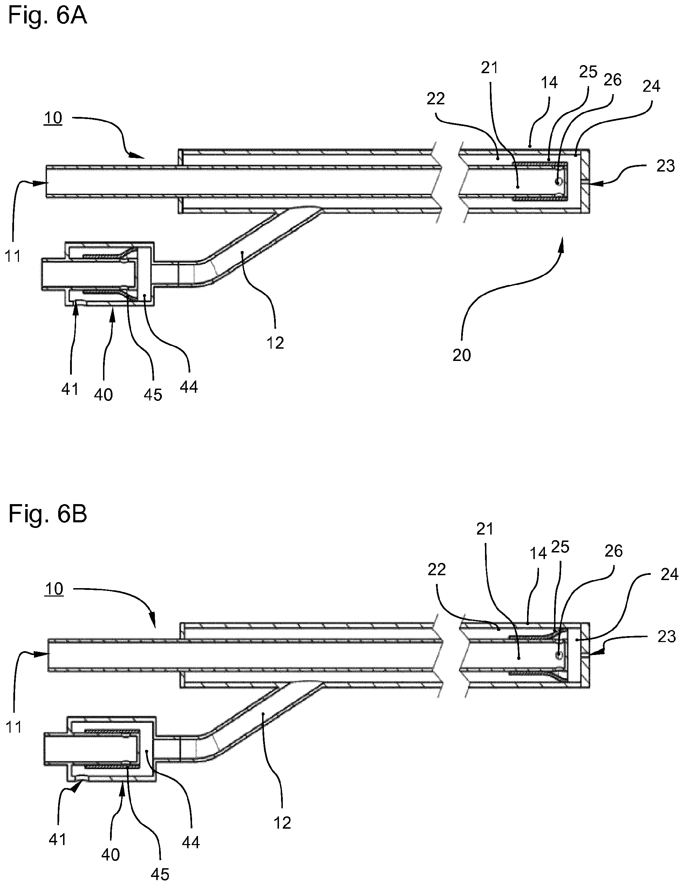

Die Fig. 6A und 6B zeigen ein weiteres erfindungsgemäßes Ausführungsbeispiel, bei dem ein elastisches Element als zweites passives Ventil verwendet wird. Im Unterschied zum Ausführungsbeispiel gemäß den Fig. 2A und 2B hat das Applikationsinstrument 10 der Fig. 6A im zweiten Zulauf eine Belüftungseinrichtung 40. Wesentliche Komponenten der Belüftungseinrichtung 40 sind eine Belüftungskammer 44, in die der zweite Zulauf 12 mündet, eine Belüftungsöffnung 41 und ein Belüftungsventil 45. Die Funktion der Belüftungsöffnung 41 wird mittels des Belüftungsventils 45 gesteuert. Die Belüftungsöffnung 41 ermöglicht eine Entlüftung des zweiten Zulaufs 12 und somit zumindest eines Abschnitts des Außenzufuhrkanals 22. Wird der zweite Zulauf 12 mit Druck beaufschlagt, schließt das Belüftungsventil 45 nach retrograd ab (Dichtwirkung zwischen zweitem Zulauf 12 und Belüftungsöffnung 41). Distal des Belüftungsventils 45 herrscht in der Belüftungskammer 44 ein Überdruck. Folglich kann das zweite Fluid in Richtung auf den Instrumentenkopf zuströmen und abgegeben werden (beispielsweise während des dritten Zeitintervalls T3). Dieser Zustand ist in der Fig. 6A gezeigt.

Fällt der Druck in dem zweiten Zulauf 12 ab, so geht das Belüftungsventil 45 in seinen Ausgangszustand über und verschließt den proximalen Teil des zweiten Zulaufs 12 gegenüber der Belüftungskammer 44 (vgl. Zustand gemäß Fig. 6B). Gleichzeitig wird der Überdruck in dem distalen Bereich des zweiten Zulaufs 12 sehr schnell über die Belüftungsöffnung 41 abgebaut. Dies führt dazu, dass der Nachlauf aus der Düse 23 sehr schnell gestoppt, im Idealfall verhindert wird, da der Strömungswiderstand der Belüftungsöffnung 41 deutlich geringer ist als derjenige der Düse 23. Insofern kann die Druckflanke des abgegebenen und gepulsten Fluidstrahls sehr steil abfallen. Da das Wechselventil 25 in diesem Zustand den Außenzufuhrkanal 22 verschließt, entweichen nur sehr geringe Mengen des zweiten Fluids durch die Belüftungsöffnung 41. Erfindungsgemäß ist es denkbar, eine Vorrichtung zum Aufnehmen der aus der Belüftungsöffnung austretenden Substanz vorzusehen und die Substanz ggf. rückzugewinnen. Erfindungsgemäß können natürlich auch mehrere Belüftungsöffnungen 41 vorgesehen sein.

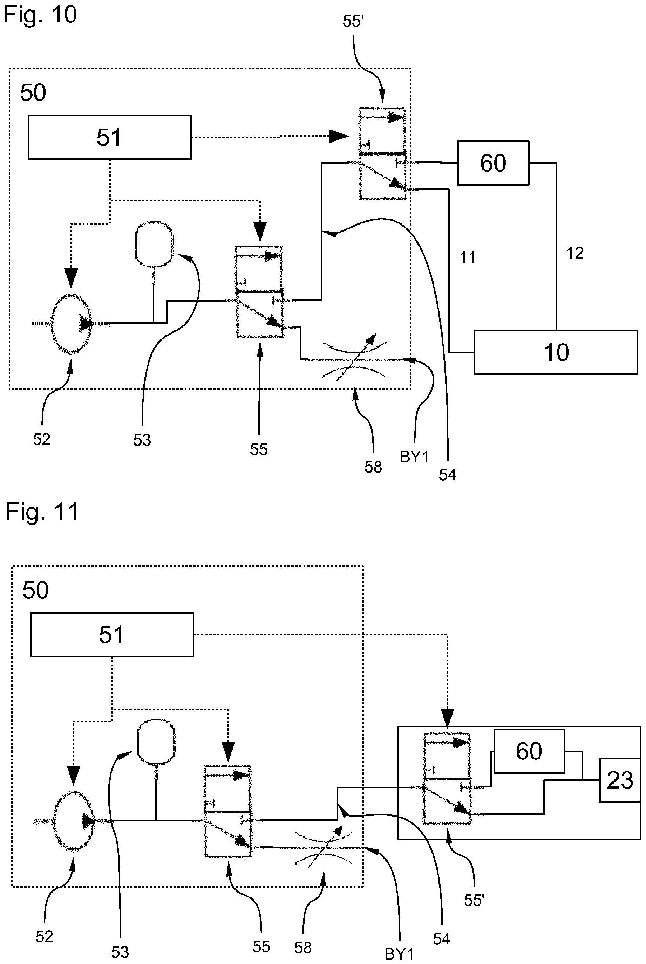

In einem anderen Ausführungsbeispiel handelt es sich bei dem Belüftungsventil 45 nicht um ein passives sondern um ein aktives Ventil bzw. ein Steuerventil. Beispielsweise kann im Handgriff des Applikationsinstruments 10 ein Magnetventil vorgesehen sein, das die Funktion des Belüftungsventils 45 erfüllt. Dieses Magnetventil kann von einer Versorgungseinrichtung 50 (vgl. Fig. 10) gesteuert werden.

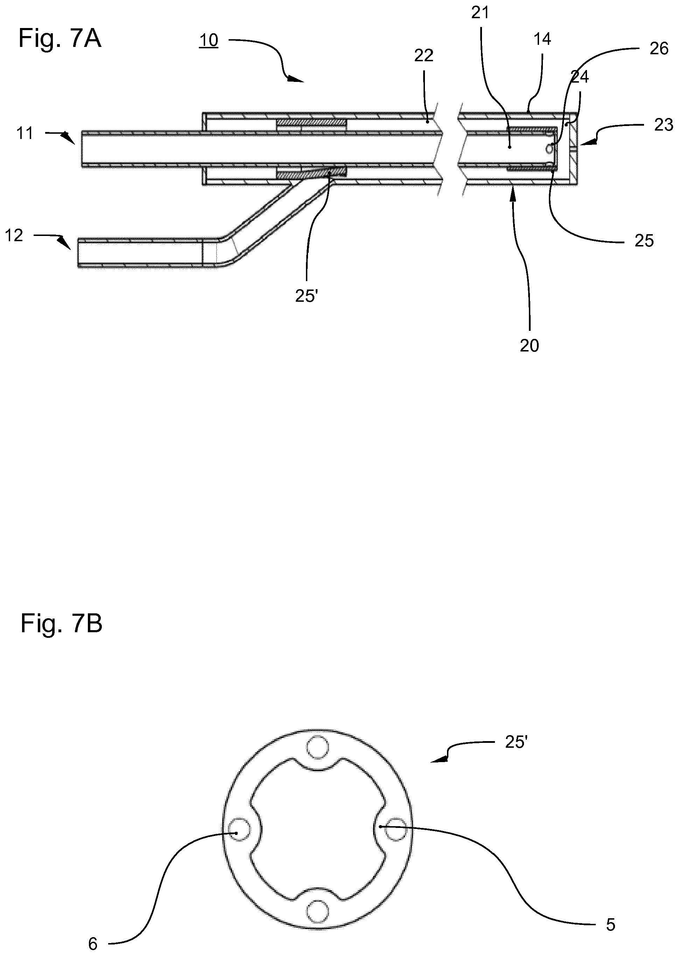

Die Fig. 7A zeigt eine Ausführungsform des Applikationsinstruments 10, die in ihrer Funktionsweise der der Fig. 3A und 3B ähnlich ist. Das Rückschlagventil 25' wird durch ein elastisches Element an einer Mündung des zweiten Zulaufs 12 in den Außenzufuhrkanal 22 gebildet. Das elastische Element ist derart ausgelegt, dass sich in einem nicht dargestellten Ausgangszustand eine Pressung zum Verschluss des zweiten Zulaufs 12 ergibt. Die dadurch erreichte Spannung erzeugt im drucklosen Zustand eine Dichtwirkung. Wird der zweite Zulauf 12 mit Druck beaufschlagt, verformt sich das elastische Element (vgl. Darstellung der Fig. 7A) und öffnet so die fluide Verbindung zu dem Außenzufuhrkanal 22. Nimmt der Druck in dem zweiten Zulauf 12 wieder ab, bringen die Rückstellkräfte das elastische Element nach Unterschreitung einer spezifischen Druckschwelle in den Ausgangszustand und das Rückschlagventil 25' schließt.

In einem Ausführungsbeispiel besteht das elastische Element aus einem elastischen Schlauchabschnitt. Durch das Anbringen von Verstärkungsstrukturen, wie beispielsweise den in Fig. 7B gezeigten in Richtung der Längsachse verlaufenden Rippen 5 und/oder Verstärkungsfasern 6 aus einem relativ steifen Material, kann eine höhere Anpressung und somit eine höhere Schließkraft des Rückschlagventils 25' erreicht werden.

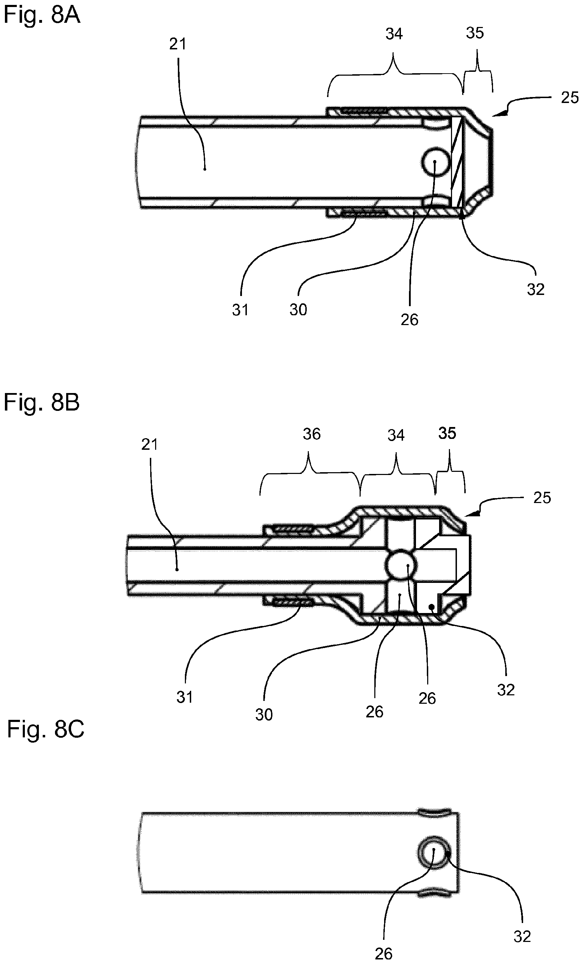

Die Fig. 8A, 8B, 8C zeigen unterschiedliche Ausführungsformen des Wechselventils 25. In einer bevorzugten Ausführungsform wird dieses durch elastisches Material ausgebildet, das an der Außenseite des Innenzufuhrkanals 21 angeordnet ist und diese mündende Seitenöffnungen 26 verschließt.

Die Ausführungsform gemäß Fig. 8A zeigt einen elastischen Schlauch 30, der abschnittsweise über das distale Ende des Innenzufuhrkanals 21 gestülpt ist. Eine Fixierung des elastischen Schlauches 30 erfolgt über einen Klemmring 31. Ein wesentlicher Aspekt der Ausführungsform gemäß Fig. 8A ist es, dass der elastische Schlauch 30 teilweise über das distale Ende des Innenzufuhrkanals 21 hinausragt. Im Endeffekt ergeben sich ein erster Abschnitt 34, an dem der elastische Schlauch auf dem Innenzufuhrkanal 21 aufliegt und ein zweiter Abschnitt 35, im Verlauf dessen sich der elastische Schlauch 30 verengt. Der elastische Schlauch 30 verläuft also über eine Dichtkante 32 mit einer besonders hohen Flächenpressung. Hierdurch wird eine bessere Dichtwirkung erzielt.

In der Ausführungsform gemäß Fig. 8B weist der Innenzufuhrkanal 21 abschnittsweise eine radial nach außen ragende Verdickung auf. Diese Verdickung ist an der Stelle angeordnet, an der sich auch die Seitenöffnungen 26 befinden. Proximal und distal der Verdickung hat der Innenzufuhrkanal 21 einen geringeren Durchmesser. Bezüglich des elastischen Schlauchs 30 ergibt sich also ein erster Abschnitt 34 mit größerem Durchmesser, ein zweiter Abschnitt 35 mit geringem Durchmesser und ein dritter Abschnitt 36 mit geringem Durchmesser. Die Durchmesser des zweiten und des dritten Abschnitts 35 und 36 können identisch sein. In einem anderen Ausführungsbeispiel (nicht dargestellt) können die Durchmesser des zweiten und des dritten Abschnitts 35 und 36 auch unterschiedliche Werte aufweisen. Durch verschiedene Durchmesser des ersten Abschnitts 34 und des zweiten 35 und/oder des dritten Abschnitts 36 ergibt sich eine Dichtkante 32, die die Schließfunktion des Wechselventils 25 verbessert. Außerdem bewirkt der größere Durchmesser im ersten Abschnitt 34 eine Vordehnung und Vorspannung des elastischen Elements, was ebenfalls eine bessere Dichtwirkung bewirkt.

Eine höhere Flächenpressung kann auch durch Zylindersegmente erzielt werden, die die Seitenöffnungen 26 des Innenzufuhrkanals 21 umgeben (vgl. Fig. 8C). Im Endeffekt bilden diese Zylindersegmente auch Dichtkanten 32, die die Schließfunktion des Ventils verbessern.

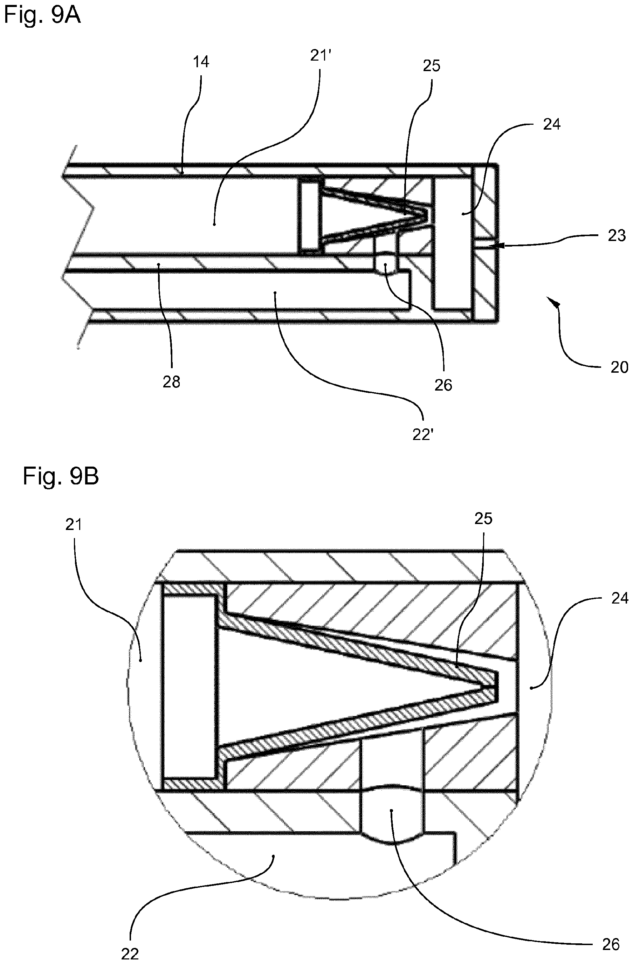

Die Fig. 9A und 9B zeigen weitere Ausführungsformen des Applikationsinstruments 10, insbesondere des Instrumentenkopfs 20. Der Sondenschaft 14 ist hier ein Mehrlumen-Schlauch (gezeigt ist ein zwellumiger Schlauch). In diesem Ausführungsbeispiel gibt es also keine koaxial zueinander angeordneten Kanäle, umfassend den Innenzufuhrkanal 21 und den Außenzufuhrkanal 22. Stattdessen verläuft ein erster Zufuhrkanal 21' für das erste Fluid parallel zu einem zweiten Zufuhrkanal 22'. Zwischen dem ersten Zulaufkanal 21' und dem zweiten Zulaufkanal 22' ist eine Trennwand 28 vorgesehen. In dem gezeigten Ausführungsbeispiel unterscheidet sich die Querschnittsfläche des ersten Zufuhrkanals 21' deutlich von der des zweiten Zufuhrkanals 22'. Beispielsweise kann der erste Zufuhrkanal 21' eine zweimal so große Querschnittsfläche wie der zweite Zufuhrkanal 22' haben. Diese sich deutlich unterscheidenden Querschnittsprofile können es gewährleisten, dass unterschiedlichen Volumenströmen (erfindungsgemäß ist der Volumenstrom des ersten Fluids größer als der des zweiten Fluids) Rechnung getragen werden. Erfindungsgemäß kann das Querschnittsprofil des ersten Zufuhrkanals 21' mehr als doppelt so groß sein als das Querschnittsprofil des zweiten Zufuhrkanals 22'.

Am distalen Ende verjüngt sich in der Ausführungsform gemäß Fig. 9A und 9B der erste Zufuhrkanal 21' und mündet dann in einen deutlich breiteren Distalspeicher 24, der in unmittelbarer fluider Verbindung mit der Düse 23 steht. Seitlich in dem verjüngenden Abschnitt des ersten Zufuhrkanals 21' mündet ungefähr mittig eine Seitenöffnung 26 des zweiten Zufuhrkanals 22'. In dem sich verjüngenden Abschnitt (= Halterungselement) ist ein elastisches Element zur Bildung eines Lippen-Rückschlagventils 25 angeordnet. Im Endeffekt ergibt sich hieraus ein bidirektionales Ventil, das die Funktion des bereits beschriebenen Wechselventils 25 übernimmt. Das Lippen-Rückschlagventil wird vorzugsweise in dem ersten Zufuhrkanal 21' durch das Halterungselement aus einem harten (nicht elastischen) Material axial fixiert. Die Innenkontur des sich verjüngenden Abschnitts entspricht weitgehend der Außenkontur des distalen Teils des Lippen-Rückschlagventils. Dabei ist die Öffnung des sich verjüngenden Abschnitts so dimensioniert, dass sich im Ausgangszustand (das elastische Element ist wie in Fig. 9A gezeigt, nicht gedehnt) ein Spalt zwischen den Lippen des elastischen Elements und der Innenwand des Halterungselements ergibt. Strömt z.B. das erste Fluid durch den ersten Zulaufkanal 21', so wird der distale Abschnitt des elastischen Elements - die Lippen des Ventils - auseinandergedehnt, so dass es oberhalb einer Druckschwelle geöffnet wird, und das erste Fluid in den Distalspeicher 24 eintreten kann. Gleichzeitig wird die untere Ventillippe durch den Druck an die Wand des sich verjüngenden Abschnitts gedrückt. Somit schließt das elastische Element die Seitenöffnung 26 aus dem zweiten Zufuhrkanal 22'. Die Druckverhältnisse des ersten Zufuhrkanals 21' sind in diesem Zustand von den Druckverhältnissen des zweiten Zufuhrkanals 22' entkoppelt. Fällt der Druck in dem ersten Zufuhrkanal 21' ab (beispielsweise nach dem ersten Zeitintervall T1), so geht das Wechselventil 25 wieder in den Ausgangszustand über, so dass die Seitenöffnung 26, wie in der Fig. 9B gezeigt, freigegeben wird. Im zweiten Zeitintervall T2 kann das zweite Fluid durch den Spalt nahezu ungehindert vorbeiströmen, so dass ein Befüllen des Distalspeichers 24 bei geringem Druck möglich ist. Da das elastische Element nun in Sperrrichtung arbeitet, wirkt es wiederum als eine Barriere zwischen dem ersten Zufuhrkanal 21' und dem zweiten Zufuhrkanal 22'. Vorzugsweise ist der Spalt derart dimensioniert, dass das zweite Fluid ungehindert, bei geringem Druck strömen kann, und andererseits das Wechselventil 25 sicher geschlossen ist, um ein Eintreten des zweiten Fluids in den ersten Zufuhrkanal 21' zu verhindern.

Die gleiche Wirkung (bidirektionale Ventilwirkung) kann auch durch die Verwendung eines innenwirkenden Ventils in Kombination mit einem Kugelventil erreicht werden. Beide Ventile sind hintereinander in einem Lumen (vorzugsweise dem Größeren) angeordnet. Das Kugelventil befindet sich dabei proximal relativ zum elastischen Element. In dieser Anordnung übernimmt jedes der Ventile die Absperrung des Fluids in je eine Richtung, während der Fluss in der jeweils anderen Richtung ungehindert erfolgt.

Alle bisher beschriebenen Ausführungsformen haben das erfindungsgemäße Ziel, unterschiedliche Druckniveaus in dem ersten Zufuhrkanal 21' bzw. Innenzufuhrkanal 21 und dem zweiten Zufuhrkanal 22' bzw. Außenzufuhrkanal 22 zu erreichen. Dazu werden die Kanäle mit Hilfe von Ventilen voneinander entkoppelt. Gleichzeitig ermöglicht der Einsatz von passiven Ventilen in der beschriebenen Form ein Unterbinden des Nachlaufs sowie die Realisierung einer Druckspeicherfunktion. Diese beiden Funktionen sind vor allem in Kombination mit der Verwendung von proximal eingesetzten aktiven Ventilen besonders vorteilhaft. Nachfolgend werden mehrere erfindungsgemäße Versorgungseinrichtungen 50 zum Betreiben der beschriebenen Applikationsinstrumente beschrieben. Erfindungsgemäß können die Versorgungseinrichtungen 50 auch mit anderen Applikationsinstrumenten 10, beispielsweise herkömmlichen Applikationsinstrumenten, verwendet werden, um die nachfolgend beschriebenen vorteilhaften Effekte zu erzielen.

Fig. 10 zeigt eine Versorgungseinrichtung 50, die über den ersten Zulauf 11 und den zweiten Zulauf 12 mit einem der beschriebenen Applikationsinstrumente 10 verbunden ist. In dem in Fig. 10 gezeigten Ausführungsbeispiel ist in dem zweiten Zulauf eine Medium-Trenneinrichtung 60 vorgesehen, so dass die Versorgungseinrichtung über unterschiedliche Anschlüsse das erste Fluid, vorzugsweise eine Kochsalzlösung, abgeben kann, wobei je nach dem gewählten Zulauf letztendlich bei dem Applikationsinstrument 10 unterschiedliche Fluide ankommen (im ersten Zulauf 11 das erste Fluid und im zweiten Zulauf 12 das zweite Fluid).

Die Versorgungseinrichtung 50 umfasst eine Steuerung, die ein Steuerverfahren implementiert, bei dem innerhalb eines Applikationszeitintervalls folgende Schritte durchgeführt werden:
- Fördern des ersten Fluids während des ersten Förderintervalls T1 mit dem hohen Druck ph in die erste Zulaufleitung 11;
- mittelbares Fördern des zweiten Fluids während des zweiten Förderintervalls T2 mit dem zweiten Druck pz in der zweiten Zulaufleitung 12 unter Nutzung der Medium-Trenneinrichtung 60; und
- Fördern des ersten Fluids während des dritten Förderintervalls T3 mit dem dritten Druck pl in der ersten Zulaufleitung.

Erfindungsgemäß kann das Steuerverfahren dazu ausgebildet sein, um auch während des vierten Förderintervalls T4 und während des fünften Förderintervalls T5 eine entsprechende Steuerstrategie anzubieten (vgl. Fig. 17).

Zur Umsetzung des Steuerverfahrens interagiert die Steuerung 51 mit einer Fluidquelle beispielsweise einer Pumpe 52, einem ersten Steuerventil 55 und einem zweiten Steuerventil 55'.

Die Pumpe 52 steht in fluider Verbindung mit einem Druckspeicher 53 der Versorgungseinrichtung 50. In dem gezeigten Ausführungsbeispiel arbeitet die Pumpe 52 kontinuierlich und ist durchflussgeregelt. Die Steuerung des ersten Steuerventils 55 das in fluider Verbindung mit dem Druckspeicher 53 steht, ermöglicht es, eine gewünschte Pulsform (Frequen, Tastgrad, effektive Pulsleistung) vorzugeben. Die Durchflussregelung der Pumpe bewirkt einen konstanten Volumenstrom des ersten Fluids innerhalb der Versorgungseinrichtung 50 unabhängig von der Schaltstellung des ersten Steuerventils 55.

Bei dem ersten Steuerventil 55 handelt es sich vorzugsweise um ein 3/2-Wegeventil, das im bestromten Zustand eine fluide Verbindung zwischen Druckspeicher 53 und einem zweiten Steuerventil 55' über eine Druckleitung 54 herstellt. Das erste Steuerventil 55 dient im Wesentlichen dazu, ein gewünschtes Druckniveau aufzubauen, während das zweite Steuerventil 55' das vorgegebene Druckniveau an den ersten Zulauf oder den zweiten Zulauf 12 anlegt.

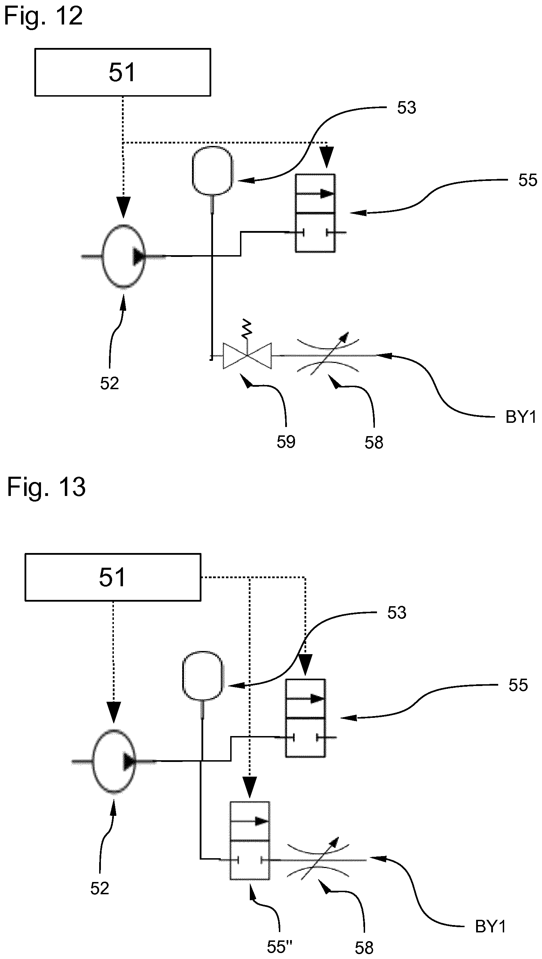

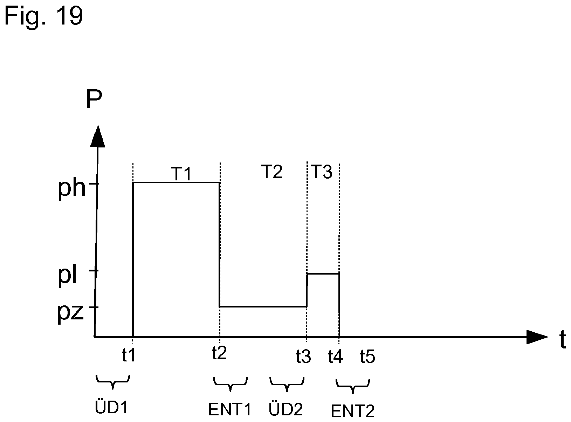

Im stromlosen Fall (vgl. Darstellung gemäß Fig. 10) des ersten Steuerventils 55 besteht eine fluide Verbindung zwischen dem Druckspeicher 53 - somit auch mit der Pumpe 52 - und einer ersten Bypassleitung BY1. Über die Bypassleitung BY1 wird der Fluidstrom abgeleitet, so dass kein unerlaubter Betriebszustand für die Pumpe 52 eintritt. Vorzugsweise ist die erste Bypassleitung BY1, wie in Fig. 10 gezeigt, mit einem Drosselventil 58 ausgestattet, das dafür sorgt, dass ein bestimmtes Druckniveau in dem Systemabschnitt vor dem ersten Steuerventil 55 gehalten wird. Zur Einstellung dieses Druckniveaus kann manuell oder über die Steuerung 51 (vgl. Fig. 13) ein hydraulischer Widerstand am Drosselventil 58 eingestellt werden. In dem in Fig. 10 gezeigten Ausführungsbeispiel ist der Widerstand voreingestellt. In dem in Fig. 10 gezeigten Zustand stellt sich in dem Druckspeicher 53 ein Druckniveau ein, das durch das Drosselventil 58 vorgegeben ist. Sobald dieses Druckniveau über das erste Steuerventil 55 an einen der beiden Zuläufe 11, 12 angelegt wird, steigt der Druck in dem jeweiligen Zulauf sprungartig an. Insofern kann ein Fluidpuls mit einer steilen Flanke abgegeben werden. Erfindungsgemäß ist es so möglich, während einer Bypassphase ÜD1, ÜD2 (vgl. Fig. 19) ein Druckniveau p'max zu erreichen, das über dem gewünschten Druck, beispielsweise dem ersten Druck ph oder dem dritten Druck pl, liegt. Diese Drücke werden vorzugsweise über die Förderleistung der Pumpe 52 eingestellt. Diese Drucküberhöhung führt dazu, dass sich der Druckimpuls sehr schnell in den Leitungen ausbreitet. Insofern kann an der Düse 23 eine sehr steile Pulsflanke erreicht werden. Des Weiteren kann die Drucküberhöhung Druckverluste in den Zuläufen 11, 12 kompensieren. Die Drucküberhöhung muss jedoch so gewählt werden, dass zwar ein schneller Kraftanstieg erreicht wird, der gewünschte Druck an der Düse 23 aber nicht überschritten wird.

Ausgehend von dem ersten Steuerventil 55 breitet sich der Druck im bestromten Zustand (nicht dargestellt) über die Druckleitungen 54 hin zu dem zweiten Steuerventil 55' aus. In dem beschriebenen Ausführungsbeispiel wählt das zweite Steuerventil 55' einen Zulauf 11, 12 aus.

In einem anderen Ausführungsbeispiel kann der Effekt der Drucküberhöhung genutzt werden, um eine initiale Perforation des biologischen Gewebes als vorbereitender Schritt für den nachfolgenden Substanzeintrag durchzuführen. In diesem Ausführungsbeispiel generiert die Versorgungseinrichtung also ein steil ansteigendes Druckprofil, das über die Zeit abfällt. Das zweite Steuerventil 55' wird so geschaltet, dass über den Verlauf der abfallenden Druckflanke erst ein Perforieren des Gewebes (erstes Zeitintervall T1), dann ein Füllen des Distalspeichers 24 (zweites Zeitintervall T2) und letztendlich noch das Eintragen der Substanz (drittes Zeitintervall T3) erfolgt.

In einem weiteren Ausführungsbeispiel (vgl. Fig. 11) ist das zweite Steuerventil 55' in das Instrument 10 integriert. Vorzugsweise erfolgt eine Steuerung des zweiten Steuerventils 55' jedoch nach wie vor über die Steuerung 51. In einem weiteren bevorzugten Ausführungsbeispiel ist zusätzlich auch das erste Steuerventil 55 in das Applikationsinstrument integriert. Vorzugsweise erfolgt eine entsprechende Integration in einen Handgriff des Applikatorinstruments 10. Hierdurch kann vermieden werden, dass die Druckpulse durch lange und/oder elastische Zuleitungen gedämpft werden. Gemäß einem Aspekt der vorliegenden Erfindung erfolgt eine Anordnung der notwendigen Steuerventile 55, 55' möglichst nah am oder innerhalb des Applikationsinstruments 10.

In einer Ausführungsform wird die Anordnung des ersten Steuerventils 55 so gewählt, dass dieses im stromlosen Zustand die Verbindung zwischen der Druckleitung 54 und der Pumpe 52 absperrt. Die Druckleitung 54 ist also während der Bypassphasen ÜD1, ÜD2 druckfrei, bzw. mit einem Restdruck beaufschlagt. Diese Anordnung hat zwei Vorteile: Erstens muss das erste Steuerventil 55 nur während der Aktivierung zur Abgabe einer Pulsfolge jeweils nur kurzzeitig mit Strom beaufschlagt werden. Zweitens steht das durch das Drosselventil 58 eingestellte Druckniveau bereits beim ersten abgegebenen Puls zur Verfügung. Fig. 12 veranschaulicht eine andere Ausführungsform zur effektiven Generierung von Fluidpulsen. In dieser Ausführungsform wird als erstes Steuerventil 55 ein 2-Wegeventil eingesetzt. Wie auch in der vorab beschriebenen Ausführungsform besteht eine fluide Verbindung zwischen der Pumpe 52 und dem Druckspeicher 53. Des Weiteren besteht eine fluide Verbindung von dem Druckspeicher zu dem ersten Steuerventil 55 in Form eines 2/2-Wegeventils.

Des Weiteren gibt es eine fluide Verbindung von dem Druckspeicher 53 hin zu einem Überdruckventil 59 im ersten Bypass, dem ein Drosselventil 58 nachgeschaltet ist. Das 2/2-Wegeventil dient dazu, einen Wassserstrahl-Impuls mit definierter Dauer abzugeben, während das Überdruckventil 59 die Erzeugung eines gewünschten Druckniveaus während der Bypassphasen, ÜD1 ÜD2 ermöglicht. Dazu kann das Überdruckventil 59 so eingestellt werden, dass dieses beim Erreichen eines bestimmten Drucks die erste Bypassleitung freigibt, so dass sich der Druck abbauen kann. Das Überdruckventil 59 kann eine Regler-Funktion übernehmen, die vorzugsweise von der Steuerung 51 gesteuert wird. In einer anderen Ausführungsvariante kann die Druck-Druckfluss-Kennlinie des Überdruckventils 59 so ausgelegt werden, dass beim Durchströmen des Ventils ein gewisser Druck an dem Ventil abfällt. In einer Ausführungsform wird auf das Überdruckventil 59 gänzlich verzichtet.

Fig. 13 zeigt ein weiteres Ausführungsbeispiel, wobei zwei 2/2-Wegeventile eingesetzt werden. Im Endeffekt wird das erste Steuerventil 55 als 2/2-Wegeventil ausgelegt und dient nach wie vor dazu, die Weitergabe des in dem System bestehenden Drucks an das Applikationsinstrument 10 zu steuern. Ein drittes Steuerventil - ebenfalls ein 2/2-Wegenventil - erlaubt eine Druckeinstellung innerhalb des Systems indem es den ersten Bypass BY1 freigibt oder verschließt. Das dritte Steuerventil 55" kann maßgeblich dazu beitragen, einen gewissen Überdruck aufzubauen.

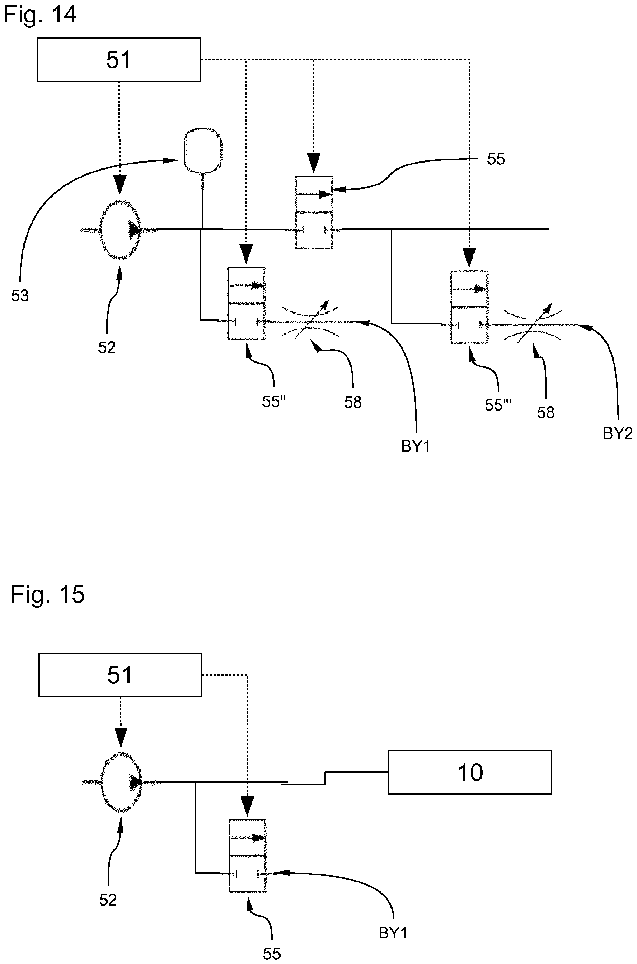

Um nach der Abgabe eines Impulses die Abfallszeit zu reduzieren, kann auf der flussabgewandten Seite des ersten Steuerventils eine weitere Bypassleitung BY2 vorgesehen sein. Fig. 14 zeigt eine entsprechende Ausführungsform der Ventilanordnung in einem Ausgangszustand wobei die Ventile entsprechend dem Applikationsschritt gesteuert werden. Ein viertes Steuerventil 55"' ermöglicht gezielt und schnell, den vorhandenen Druck in den Zuläufen 11, 12' abzubauen. Diese Ausführungsform ermöglicht es, Entlüftungsphasen ENT1, ENT2 umzusetzen, wie diese in der Fig. 19 gezeigt sind. Mit der in der Fig. 14 gezeigten Ausführungsform ist es also möglich, in einer ersten Bypassphase ÜD1 einen Überdruck aufzubauen und diesen dann gezielt an das Applikationsinstrument 10 abzugeben, so dass an dessen Düse 23 ein Puls mit einer möglichst steilen Flanke abgegeben wird. Danach treibt die Pumpe 52 das Fluid weiter an, um den ersten Druck während des ersten Zeitintervalls T1 aufrechtzuerhalten. Danach wird das erste Steuerventil 55 geschlossen, um den Puls zu beenden (Zeitpunkt t2). Gleichzeitig wird das vierte Steuerventil 55"' geöffnet, um eine fluide Verbindung zu der zweiten Bypassleitung BY2 zu schaffen. Hierdurch wird die erste Entlüftungsphase ENT1 implementiert. Die Abfallzeit ist sehr gering und führt zu einem unmittelbaren Druckabbau beispielsweise im Distalspeicher 24. In ähnlicher Weise kann verfahren werden, um während des dritten Zeitintervalls T3 - Applikation der Suspension - eine möglichst steile Pulsflanke am Anfang (Bypassphase ÜD2) und am Ende (Entlüftungsphase ENT2) zu erzielen.

Fig. 15 zeigt ein Ausführungsbeispiel, bei dem ein 2/2-Wegeventil als erstes Steuerventil 55 verwendet wird. Das 2/2-Wegeventil ist die Bypassleitung BY1 integriert, während das Applikationsinstrument 10 direkt mit der Pumpe verbunden ist. Ein Vorteil dieses Aufbaus besteht darin, dass auch hier Entlüftungsphasen ENT1, ENT2 für einen schnellen Druckabfall implementiert werden können. Gegenüber den bisher beschriebenen Ausführungsformen ist diese Ausführungsform sehr einfach und robust.

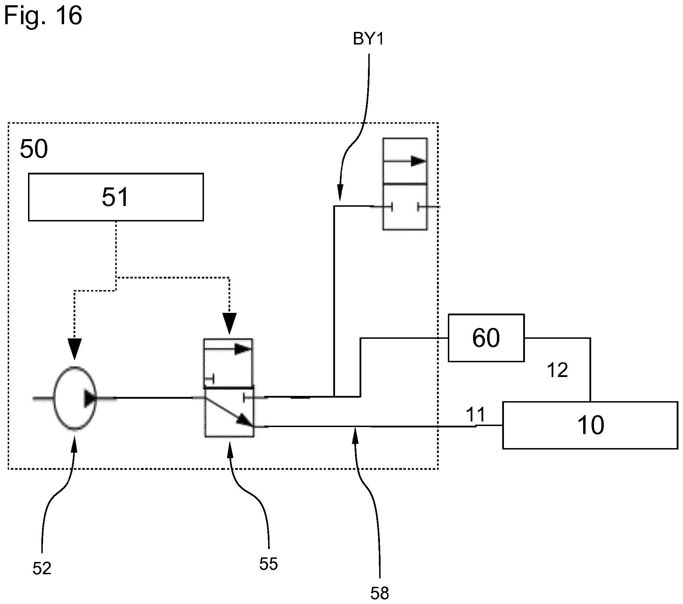

Fig. 16 zeigt ein weiteres Ausführungsbeispiel der erfindungsgemäßen Versorgungseinrichtung 50. Hier werden zwei 3/2-Wegeventile eingesetzt, um die bereits beschriebene Funktionalität umzusetzen.

Die beschriebenen aktiven Ventile bzw. Steuerventile können einen elektromagnetischen Antrieb oder einen anderen im Stand der Technik bekannten Antrieb besitzen. Beispielsweise können Piezo-Aktuatoren, eine pneumatische Antriebseinheit oder Ähnliches verwendet werden. Außerdem können die Ausführungsformen in beliebiger Weise miteinander kombiniert werden. Zur Umsetzung der Erfindung können Nadelventile, Membranventile, Wippenventile und andere eingesetzt werden. Zur Umsetzung des beschriebenen 2/2-Wegeventils kann beispielsweise ein Klemmventil eingesetzt werden, das aufgrund seiner Sterilisierbarkeit bevorzugt ist.

Fig. 17 und Fig. 18 zeigen unterschiedliche Druckverläufe, mit jeweils einem Puls für die Perforation des Gewebes (=erstes Zeitintervall T1) und zwei Pulsen für die Applikation der Substanz (=drittes und fünftes Zeitintervall T3 und T5). Das zweite Zeitintervall T2 wird jeweils genutzt, um den Distalspeicher 24 zu füllen. Gemäß Fig. 17 erfolgt ein Nachfüllen des Distalspeichers 24 in dem vierten Zeitintervall T4. Gemäß Fig. 18 unterbleibt ein entsprechendes Nachfüllen.

Fig. 19 zeigt die zeitliche Anordnung der Bypassphasen BY1, BY2 und der Entlüftungsphase ENT1, ENT2 relativ zu den Zeitintervallen T1 bis T5. Die erste Bypassphase endet mit dem Beginn des ersten Zeitintervalls T1 zum Zeitpunkt t1. Die erste Entlüftungsphase ENT1 beginnt am Ende des ersten Zeitintervalls T1 zum Zeitpunkt t2. Im Intervall zwischen t2 und t3 beginnt die zweite Bypassphase BY2, die zum Zeitpunkt t3 mit dem Beginn des dritten Zeitintervalls T3 endet. Am Ende des dritten Zeitintervalls T3 beginnt zum Zeitpunkt t4 die zweite Entlüftungsphase ENT2 die zum Zeitpunkt t5 endet.

### Bezugszeichenliste

- 1: Mucosa
- 2: Muscularis
- 3: Lamina propria
- 4: Circular muscule
- 5: Rippe
- 6: Verstärkungsfaser
- 10: Applikationsinstrument
- 11: Erster Zulauf, erste Zulaufleitung
- 12: Zweiter Zulauf, zweite Zulaufleitung
- 14: Sondenschaft
- 20: Instrumentenkopf
- 21: Innenzufuhrkanal
- 21': Erster Zufuhrkanal
- 22: Außenzufuhrkanal
- 22': Zweiter Zufuhrkanal
- 23: Austrittsöffnung, Düse
- 24: Distalspeicher
- 25: Wechselventil
- 25': Rückschlagventil
- 26: Seitenöffnung
- 28: Trennwand
- 30: Elastischer Schlauch
- 31: Klemmring
- 32: Dichtkante
- 34: Erster Abschnitt
- 35: Zweiter Abschnitt
- 36: Dritter Abschnitt
- 40: Belüftungseinrichtung
- 41: Belüftungsöffnung
- 44: Belüftungskammer
- 45: Belüftungsventil
- 50: Versorgungseinrichtung
- 51: Steuerung
- 52: Pumpe
- 53: Druckspeicher
- 54: Druckleitung
- 55, 55', 55", 55"': Steuerventil
- 58: Drosselventil
- 59: Überdruckventil
- 60: Mediumtrenneinrichtung
- 100: Applikationssystem
- Ad: Außendurchmesser
- BY1, BY2: Bypassleitung
- ÜD1, ÜD2: Bypassphase
- ENT1, ENT2: Entlüftungsphase
- t1-t6: Zeitpunkt
- T1-T5: Zeitintervall
- pz, pl, ph: Druck

## Patentansprüche

1. Versorgungseinrichtung mit mindestens einem Auslass zum Anschluss eines Applikationsinstruments und mit einer Steuerung (51), die mindestens ein Ventil (55, 55', 55", 55"') steuert,
**dadurch gekennzeichnet, dass**
die Steuerung (51) das mindestens eine Ventil (55, 55', 55", 55"') derart steuert,
dass innerhalb eines Applikationszeitintervalls von weniger als 4 Sekunden:
a) ein erstes Fluid während mindestens eines ersten Förderintervalls (T1) mit einem ersten Druck (ph) in einer ersten Zulaufleitung (11);
b) ein zweites Fluid während eines zweiten, dem ersten Förderintervall (T1) zeitlich nachgelagerten Förderintervalls (T2) mit einem zweiten Druck (pz) in einer zweiten Zulaufleitung (12); und
c) das erste Fluid während mindestens eines dritten Förderintervalls (T3) mit einem dritten Druck (pl) in der ersten Zulaufleitung (11) gefördert wird.

2. Versorgungseinrichtung nach Anspruch 1,
**gekennzeichnet durch**
mindestens eine Pumpe (52), die **durch** die Steuerung (51) derart gesteuert wird, dass ein im Wesentlichen konstanter Volumenfluss des ersten Fluids, während einer Abgabephase und einer Bypassphase (ÜD1, ÜD2) erzielt wird.

3. Versorgungseinrichtung nach einem der vorhergehenden Ansprüche,
**gekennzeichnet durch**
eine Bypassleitung (BY1) zum Abführen des Fluids, wobei die Bypassleitung (BY1) ein Drosselelement, umfasst und die Steuerung (51) mindestens ein erstes Ventil (55,) derart steuert, dass zur gepulsten Abgabe des ersten Fluids an dem Auslass abwechselnd eine fluide Verbindung zwischen Pumpe (52) und einer Druckleitung (54) in einer Abgabephase und zwischen Pumpe (52) und Bypassleitung (BY1) in einer/der Bypassphase (ÜD1, ÜD2) hergestellt wird.

4. Versorgungseinrichtung nach Anspruch 3,
**dadurch gekennzeichnet, dass**
die Steuerung (51) das mindestens eine erste Ventil (55,) derart steuert, dass innerhalb des Applikationszeitintervalls mindestens eine Abgabephase und mindestens eine Bypassphase (ÜD1, ÜD2) implementiert wird.

5. Versorgungseinrichtung nach Anspruch 3 oder 4,
**dadurch gekennzeichnet, dass**
das mindestens eine erste Ventil (55,) ein elektrisches Ventil ist, das derart angeordnet und ausgebildet ist, dass in einer bestromten Phase eine fluide Verbindung zwischen Pumpe (52) und Druckleitung (Abgabephase) besteht.

6. Versorgungseinrichtung nach einem der Ansprüche 3 bis 5,
**dadurch gekennzeichnet, dass**
das/ein Drosselelement derart eingestellt ist und/oder die Steuerung (51) ein drittes Ventil (55") an der/einer Bypassleitung derart einstellt, dass an dem dritten Ventil (55") ein Bypassdruck (p'max) anliegt, der um mindestens 50% oder um mindestens 100% des dritten Drucks (pl) größer ist; und/oder
das Drosselelement derart eingestellt ist und/oder die Steuerung (51) das dritte Ventil (55") an der Bypassleitung (BY1) derart einstellt, dass ein Bypassdruck (p'max) anliegt, der größer ist als der erste Druck (ph), nämlich um mindestens 5% oder um mindestens 10% des ersten Drucks (ph).

7. Versorgungseinrichtung nach einem der vorhergehenden Ansprüche,
**dadurch gekennzeichnet, dass**
die Steuerung (51) mindestens ein zweites Ventil (55') derart steuert, dass abwechselnd eine fluide Verbindung zwischen der/einer Druckleitung (54) und der ersten Zulaufleitung (11) und zwischen der Druckleitung (54) und der zweiten Zulaufleitung (12) hergestellt wird.

8. Versorgungseinrichtung nach einem der vorhergehenden Ansprüche,
**gekennzeichnet durch**
eine (weitere) Bypassleitung (BY2), die über ein **durch** die Steuerung (51) gesteuertes viertes Ventil (55"'), nämlich über ein 2/2-Wegeventil, mit der/einer Druckleitung (54) in fluide Verbindung bringbar ist, um die Druckleitung (54) in einer Entlüftungsphase (ENT1, ENT2) zu entlüften.

9. Versorgungseinrichtung nach Anspruch 8,
**dadurch gekennzeichnet, dass**
die Steuerung (51) das vierte Ventil (55"') derart steuert, dass innerhalb des Applikationszeitintervalls mindestens eine Entlüftungsphase (ENT1, ENT2), implementiert wird.

10. Applikationssystem, umfassend:
- ein Applikationsinstrument; und
- eine Versorgungseinrichtung (50) gemäß einem der Ansprüche 1 bis 9,
wobei mindestens eines der Ventile (55, 55', 55", 55"') im Applikationsinstrument angeordnet ist und über mindestens eine Steuerleitung von der Steuerung (51) gesteuert wird.

11. Computerlesbarer Speicher mit Instruktionen zur Implementierung eines Steuerverfahrens zum Betreiben einer Versorgungseinrichtung (50), wenn die Instruktionen auf einer Recheneinheit ausgeführt werden, wobei das Steuerverfahren die folgenden Schritte umfasst:
a) Aktivieren einer ersten Pumpe (52) derart, dass in einer Bypassphase (ÜD1, ÜD2) ein Bypassdruck (p'max) aufgebaut wird;
b) Öffnen mindestens eines Ventils derart, dass in einer Abgabephase ein erstes Fluid mit dem Bypassdruck (p'max) in eine Druckleitung (54) abgegeben wird;
c) Betreiben der Pumpe (52) derart, dass nach der Bypassphase (ÜD1, ÜD2) und während der Abgabephase ein Druck in der Druckleitung (54) vorliegt, der geringer ist als der Bypassdruck (p'max).

12. Computerlesbarer Speicher nach Anspruch 11,
**dadurch gekennzeichnet, dass** das Steuerverfahren umfasst:
d) ein Schalten mindestens eines Ventils derart, dass das erste Fluid zeitweise ein zweites Fluid antreibt.

## Claims

1. Supply device having at least one outlet for connection of an application instrument, and having a controller (51), which controls at least one valve (55, 55', 55", 55"'),
**characterized in that**
the controller (51) controls the at least one valve (55, 55', 55", 55"') such
that, within an application time interval of less than 4 seconds:
a) a first fluid is delivered, during at least a first delivery interval (T1), at a first pressure (ph) in a first inflow line (11);
b) a second fluid is delivered, during a second delivery interval (T2), which follows the first delivery interval (T1), at a second pressure (pz) in a second inflow line (12); and
c) the first fluid is delivered, during at least a third delivery interval (T3), at a third pressure (pl) in the first inflow line (11).

2. Supply device according to Claim 1,
**characterized by**
at least one pump (52), which is controlled by the controller (51) such that an essentially constant volume flow of the first fluid is achieved during a discharge phase and a bypass phase (ÜD1, ÜD2).

3. Supply device according to either of the preceding claims,
**characterized by**
a bypass line (BY1) for channelling away the fluid, wherein the bypass line (BY1) comprises a throttle element, and the controller (51) controls at least one first valve (55) such that, for pulsed discharge of the first fluid at the outlet, in alternating fashion a fluid connection is established between the pump (52) and pressure line (54) in a discharge phase and between the pump (52) and bypass line (BY1) in a/the bypass phase (ÜD1, ÜD2).

4. Supply device according to Claim 3,
**characterized in that**
the controller (51) controls the at least one first valve (55) such that, within the application time interval, at least one discharge phase and at least one bypass phase (ÜD1, ÜD2) are implemented.

5. Supply device according to Claim 3 or 4,
**characterized in that**
the at least one first valve (55) is an electric valve which is arranged, and designed, such that, in an energized phase, there is a fluid connection between the pump (52) and pressure line (discharge phase).

6. Supply device according to one of Claims 3 to 5,
**characterized in that**
the/a throttle element is set, and/or the controller (51) sets a third valve (55") on the/a bypass line, such that the bypass pressure (p'max) present at the third valve (55") is greater than the third pressure (pl) by at least 50% or by at least 100%; and/or
the throttle element is set, and/or the controller (51) sets the third valve (55") on the bypass line (BY1), such that the bypass pressure (p'max) present is greater than the first pressure (ph), that is to say is greater than the first pressure (ph) by at least 5% or by at least 10%.

7. Supply device according to one of the preceding claims,
**characterized in that**
the controller (51) controls at least one second valve (55') such that in alternating fashion a fluid connection is established between the/a pressure line (54) and the first inflow line (11) and between the pressure line (54) and the second inflow line (12).

8. Supply device according to one of the preceding claims,
**characterized by**
a (further) bypass line (BY2), which can be brought into fluidic connection with the/a pressure line (54) via a fourth valve (55"'), that is to say via a 2/2-way valve, controlled by the controller (51), in order to vent the pressure line (54) in a venting phase (ENT1, ENT2).

9. Supply device according to Claim 8,
**characterized in that**
the controller (51) controls the fourth valve (55"') such that, within the application time interval, at least one venting phase (ENT1, ENT2) is implemented.

10. Application system comprising:
- an application instrument; and
- a supply device (50) according to one of Claims 1 to 9,
wherein at least one of the valves (55, 55', 55", 55"') is arranged in the application instrument and is controlled by the controller (51) via at least one control line.

11. Computer-readable memory with instructions for implementing a control method for operating a supply device (50) when the instructions are executed on a computing unit, wherein the control method comprises the following steps:
a) activating a first pump (52) such that, in a bypass phase (ÜD1, ÜD2), a bypass pressure (p'max) is built up;
b) opening at least one valve such that, in a discharge phase, a first fluid is discharged at the bypass pressure (p'max) into a pressure line (54); and
c) operating the pump (52) such that, following the bypass phase (ÜD1, ÜD2) and during the discharge phase, the pressure present in the pressure line (54) is smaller than the bypass pressure (p'max).

12. Computer-readable memory according to Claim 11, **characterized in that** the control method also comprises:
d) switching at least one valve such that the first fluid, at times, drives a second fluid.

## Revendications

1. Dispositif d'alimentation avec au moins une sortie pour le raccordement d'un instrument d'application et avec une commande (51), qui commande au moins une soupape (55, 55', 55", 55"'), **caractérisé en ce que** la commande (51) commande ladite au moins une soupape (55, 55', 55", 55"') de telle manière qu'à l'intérieur d'un intervalle de temps d'application de moins de 4 secondes:
a) un premier liquide soit transporté pendant au moins un premier intervalle de transport (T1) avec une première pression (ph) dans une première conduite d'arrivée (11);
b) un deuxième liquide soit transporté pendant un deuxième intervalle de transport (T2) succédant temporellement au premier intervalle de transport (T1), avec une deuxième pression (pz) dans une deuxième conduite d'arrivée (12); et
c) le premier liquide soit transporté pendant au moins un troisième intervalle de transport (T3) avec une troisième pression (pl) dans la première conduite d'arrivée (11).

2. Dispositif d'alimentation selon la revendication 1, **caractérisé par** au moins une pompe (52), qui est commandée par la commande (51) de telle manière qu'un flux volumique essentiellement constant du premier liquide soit atteint pendant une phase de décharge et une phase de dérivation (ÜD1, ÜD2).

3. Dispositif d'alimentation selon l'une quelconque des revendications précédentes, **caractérisé par** une conduite de dérivation (BY1) pour l'évacuation du liquide, dans lequel la conduite de dérivation (BY1) comprend un élément d'étranglement et la commande (51) commande au moins une première soupape (55) de telle manière qu'une communication liquide soit établie en alternance entre la pompe (52) et une conduite de refoulement (54) dans une phase de décharge et entre la pompe (52) et la conduite de dérivation (BY1) dans une/la phase de dérivation (ÜD1, ÜD2) pour la décharge pulsée du premier liquide à la sortie.

4. Dispositif d'alimentation selon la revendication 3, **caractérisé en ce que** la commande (51) commande ladite au moins une première soupape (55) de telle manière qu'à l'intérieur de l'intervalle de temps d'application au moins une phase de décharge et au moins une phase de dérivation (ÜD1, ÜD2) soient exécutées.

5. Dispositif d'alimentation selon la revendication 3 ou 4, **caractérisé en ce que** ladite au moins une première soupape (55) est une soupape électrique, qui est disposée et configurée de telle manière qu'il existe dans une phase activée une communication liquide entre la pompe (52) et une conduite de refoulement (phase de décharge).

6. Dispositif d'alimentation selon l'une quelconque des revendications 3 à 5, **caractérisé en ce que** le/un élément d'étranglement est réglé de telle manière et/ou la commande (51) règle une troisième soupape (55") au niveau de la/une conduite de dérivation de telle manière qu'il règne au niveau de la troisième soupape (55") une pression de dérivation (p'max), qui est plus élevée d'au moins 50 % ou d'au moins 100 % de la troisième pression (pl); et/ou
l'élément d'étranglement est réglé de telle manière et/ou la commande (51) règle la troisième soupape (55") au niveau de la conduite de dérivation (BY1) de telle manière qu'il règne une pression de dérivation (p'max), qui est plus élevée que la première pression (ph), à savoir d'au moins 5 % ou d'au moins 10 % de la première pression (ph).

7. Dispositif d'alimentation selon l'une quelconque des revendications précédentes, **caractérisé en ce que** la commande (51) commande au moins une deuxième soupape (55') de telle manière qu'une communication liquide soit établie en alternance entre la/une conduite de refoulement (54) et la première conduite d'arrivée (11) et entre la conduite de refoulement (54) et la deuxième conduite d'arrivée (12).

8. Dispositif d'alimentation selon l'une quelconque des revendications précédentes, **caractérisé par** une (autre) conduite de dérivation (BY2), qui peut être mise en communication liquide avec la/une conduite de refoulement (54) par une quatrième soupape (55"') commandée par la commande (51), à savoir une soupape à 2/2 voies, afin de ventiler la conduite de refoulement (54) dans une phase de ventilation (ENT1, ENT2).

9. Dispositif d'alimentation selon la revendication 8, **caractérisé en ce que** la commande (51) commande la quatrième soupape (55"') de telle manière qu'au moins une phase de ventilation (ENT1, ENT2) soit exécutée à l'intérieur de l'intervalle de temps d'application.

10. Système d'application, comprenant:
- un instrument d'application; et
- un dispositif d'alimentation (50) selon l'une quelconque des revendications 1 à 9,
dans lequel au moins une des soupapes (55, 55', 55", 55"') est disposée dans l'instrument d'application et est commandée par la commande (51) via au moins une conduite de commande.

11. Mémoire adressable par ordinateur avec des instructions pour l'exécution d'un procédé de commande en vue du fonctionnement d'un dispositif d'alimentation (50), lorsque les instructions sont exécutées sur une unité informatique, dans laquelle le procédé de commande comprend les étapes suivantes:
a) activer une première pompe (52) de telle manière qu'une pression de dérivation (p'max) soit établie dans une phase de dérivation (ÜD1, ÜD2);
b) ouvrir au moins une soupape de telle manière que dans une phase de décharge un premier liquide soit déchargé dans une conduite de refoulement (54) avec la pression de dérivation (p'max);
c) faire fonctionner la pompe (52) de telle manière qu'après la phase de dérivation (ÜD1, ÜD2) et pendant la phase de décharge, il règne dans la conduite de refoulement (54) une pression qui est inférieure à la pression de dérivation (p'max).

12. Mémoire adressable par ordinateur selon la revendication 11, **caractérisé en ce que** le procédé de commande comprend l'opération suivante:
d) commuter au moins une soupape, de telle manière que le premier liquide entraîne temporairement un deuxième liquide.
